(19) Europäisches Patentamt
European Patent Office
Office européen des brevets

(11) **EP 3 118 323 A1**

(12) **EUROPEAN PATENT APPLICATION**

(43) Date of publication:
**18.01.2017 Bulletin 2017/03**

(51) Int Cl.:
***C12Q 1/68*** (2006.01)

(21) Application number: **15176401.6**

(22) Date of filing: **13.07.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Cartagenia N.V.**
**3001 Leuven (BE)**

(72) Inventors:
- **Allemeersch, Joke**
  **3200 Aarschot (BE)**
- **Devogelaere, Benoit**
  **1800 Vilvoorde (BE)**

(74) Representative: **Brantsandpatents bvba**
**Pauline Van Pottelsberghelaan 24**
**9051 Ghent (BE)**

(54) **SYSTEM AND METHODOLOGY FOR THE ANALYSIS OF GENOMIC DATA OBTAINED FROM A SUBJECT**

(57)    The current invention describes a method for determining the presence or absence of a fetal chromosomal aneuploidy in a pregnant female, the method comprising the calculation of a parameter p from sequences obtained from a biological sample from said pregnant female. The current invention equally provides a method for determining the fetal fraction of said sample.

**EP 3 118 323 A1**

Printed by Jouve, 75001 PARIS (FR)

**Description**

**Technical Field**

The invention pertains to a method and system for the analysis of genomic data from a subject. In particular, the current invention relates to a method allowing the determination of the presence of a fetal aneuploidy in a sample in an essentially non-invasive manner.

**INTRODUCTION**

The availability of high-throughput DNA sequencing technologies has enabled nearly comprehensive investigations into the number and types of sequence variants possessed by individuals in different populations and with different diseases. Whole genome sequencing may become a relatively routine procedure in the near future as high-throughput sequencing costs and efficiency continue to improve. In fact, as costs continue to decline, high-throughput sequencing is expected to become a commonly used tool, not only in human phenotype based sequencing projects, but also as an effective tool in forward genetics applications in model organisms, and for the diagnosis of diseases previously considered to be idiopathic.

Once a sequence is obtained, an effort is made to identify the location and character of those portions of a sequence that differ from one or more "standard" reference sequences, with each difference commonly referred to as a variant. This can help identify those portions of an individual's genome that could potentially contribute to a clinical condition or other trait of the individual. For example, it is common to compare the sequence of a particular individual with reference human genome sequences maintained by the University of California, Santa Cruz, and create a list of the variants that exist between an individual's sequence and a reference sequence.

This variant list may include millions of variants, but provides little if any information on the impact any particular variant may have on gene function. Research programs around the world are continually gathering information relating particular variants to gene function, disease states, and the like. Furthermore, a variety of computational methods have been developed to deduce possible physiological effects of some types of variants based on their location on the genome and the nature of the variant, even if no laboratory biochemical or clinical studies have been undertaken on that particular variant.

Prenatal analysis of a fetus has nowadays more and more shifted towards a genomic analysis of the fetus and the parental DNA.

The presence of circulating extracellular DNA in the peripheral blood is a well-established phenomenon. It has been shown that in the case of a pregnant woman extracellular fetal DNA is present in the maternal circulation and can be detected in maternal plasma or serum. In fact, while most people in the field describe this type of DNA as fetal DNA, it is in fact placental DNA, and minor differences may occur between fetal and placental DNA due to mosaicism originating during embryogenesis. However, throughout this document, the term "fetal DNA" will be used as this is the most commonly used terminology to describe this type of DNA. Studies have shown that this circulating fetal genetic material can be used for the very reliable determination, e.g. by PCR (polymerase chain reaction) technology, of fetal genetic loci which are completely absent from the maternal genome. Examples of such fetal genetic loci are the fetal RhD gene in pregnancies at risk for HDN (hemolytic disease of the fetus and newborn) or fetal Y chromosome-specific sequences in pregnancies at risk for an X chromosome-linked disorder e.g. hemophilia or fragile X syndrome.

Fetal aneuploidy and other chromosomal aberrations affect 9 out of 1000 live births. The gold standard for diagnosing chromosomal abnormalities is karyotyping of fetal cells obtained via invasive procedures such as chorionic villus sampling and amniocentesis. These procedures impose small but potentially significant risks to both the fetus and the mother. In the past years, progression has been made to develop non-invasive screening methods for fetal chromosomal abnormalities.

Since the discovery of intact fetal cells in maternal blood, there has been intense interest in trying to use them as a diagnostic window into fetal genetics. EP2183693 and family members describe a methodology for performing a prenatal diagnosis of a fetal chromosomal aneuploidy in a biological sample obtained from the pregnant mother. The sample is randomly sequenced and the obtained sequences are used to determine a parameter which is a used to evaluate the presence or absence of a fetal aneuploidy.

US8195415 describes a method for evaluating whether a chromosome has an abnormal distribution in a sample obtained from a subject. The method includes shotgun sequencing of the DNA present in the sample, and subsequently aligning the obtained sequence tags to chromosome portions. Values are determined based on the number of alignments obtained which are then used to calculate a differential which is determinative of whether or not an abnormal distribution exists.

Bayindir et al., 2015 describes a noninvasive prenatal testing methodology based on the Z and ZZ score, whereby said Z-score is a chromosomal-wide-Z-score and the ZZ-score is calculated as the standard score of the Z-score of a given autosome in comparison with the Z-scores of remaining autosomes.

Although above mentioned methodologies have their value, the ratio of false positives and negatives remain high in the field. It is therefore a constant striving to provide methodologies that are able to lower the percentage of false positives and especially false negatives, in order to provide more accurate and robust screening.

The current invention wishes to provide a more accurate non-invasive methodology for determining whether an aneuploidy exist in a fetus, along with the determination of the fetal fraction as this is deemed to be an important quality metric to assess the risk for false-negatives in a sample.

From a broader perspective, the current invention also wishes to provide a methodology and tools to analyzing genomic data, e.g. for genomic variant annotation.

## SUMMARY OF THE INVENTION

The invention pertains to a method for determining the presence or absence of a aneuploidy according to claim 1 or any of the dependent claims. The claimed methodology allows evaluating the presence or absence of such aneuploidy on the basis of a set of reference samples. The current methodology offers a reliable and robust parameter for determining the presence of an aneuploidy. The methodology is sensitive and minimizes false positives and false negatives.

The current invention also provides for a method to determine the fetal fraction according to claim 27 and dependent claims. The method provides a straightforward and easy estimation of the fetal fraction in a sample based on the available low-coverage, random sequencing data. The latter also serves as an additional quality control for the aneuploidy detection, especially for the cases where no aneuploidy was detected (as some of these could be false-negatives, because the fetal fraction was too low to enable the detection of an aneuploidy using low-coverage sequencing).

Finally, the current methodology also provides for a computer program product according to claim 19 or 30, able to perform one or more operations according to the current invention and a report generated thereby according to claim 25.

## FIGURES

**Figure 1** shows a report according to an embodiment of the current invention, displaying calculated parameters and visual representation for chromosome 21 in a sample A, whereby said chromosome 21 was identified as abnormal by the method according to the current invention.

**Figure 2** shows a plot of the parameters obtained for all chromosomes within one sample A. Only chromosome 21 showed an aberration.

**Figure 3** shows a report according to an embodiment of the current invention, displaying calculated parameters and visual representation for chromosome 11 in sample A. No aberrations are observed.

**Figure 4** shows a plot of the calculated secondary parameters according to an embodiment of the current invention, calculated for all chromosomes in sample A. A trisomy was observed.

**Figure 5** depicts a report according to an embodiment of the current invention, displaying calculated parameters and visual representation for chromosome 16 in sample B. A trisomy was observed.

**Figure 6** shows a plot of the parameters obtained for all chromosomes within sample B. Only one chromosome, chromosome 16, showed an aberration.

**Figure 7** shows a report according to an embodiment of the current invention, displaying calculated parameters and visual representation for chromosome 1 in sample B. No trisomy was observed.

**Figure 8** shows a plot of the calculated secondary parameters according to an embodiment of the current invention, calculated for all chromosomes in sample B. A trisomy was observed.

**Figure 9** shows a report according to an embodiment of the current invention, displaying calculated parameters and visual representation for chromosome 21 in a sample A, whereby said chromosome 21 was identified as abnormal by the method according to the current invention.

**Figure 10** shows a plot of the parameters obtained for all chromosomes within one sample A. Only one chromosome showed an aberration.

**Figure 11** shows a report according to an embodiment of the current invention, displaying calculated parameters and visual representation for chromosome 11 in sample A. No aberrations are observed.

**Figure 12** depicts a report according to an embodiment of the current invention, displaying calculated parameters and visual representation for chromosome 16 in sample B. An aneuploidy was observed.

**Figure 13** shows a plot of the parameters obtained for all chromosomes within sample B. Only one chromosome 16 showed an aberration.

**Figure 14** shows a report according to an embodiment of the current invention, displaying calculated parameters and visual representation for chromosome 1 in sample B. No trisomy was observed.

**Figure 15** show a plot of a secondary parameter indicative for the success rate of the experiment obtained for all chromosomes in sample A according to an embodiment of the current example. The experiment was successful.

**Figure 16** show a plot of a secondary parameter indicative for the success rate of the experiment obtained for all chromosomes in sample B according to an embodiment of the current example. The experiment was successful.

**Figure 17** shows a plot for a chromosome in a sample, obtained by an embodiment of the current invention, indicating the presence of a polymorphic site, more particularly a Copy Number Variation that could be present in the maternal genome instead of the fetal genome.

**Figures 18 to 24** show plots for a chromosome in a sample obtained by an embodiment of the current invention. The methodology of the current invention is proven to have a higher sensitivity in view of prior art methods.

**Figure 25** shows a plot with the number of reads mapping to the Y chromosome for samples that were categorized as male, female, or of undetermined gender.

**Figure 26** shows a plot with of the X and Y-based fetal fraction estimations for a set of male pregnancies.

**Figure 27** shows the histograms of the normalized counts for a particular polymorphic site within a set of test samples.

**Figure 28 and 29** show a plot of the estimated fetal fraction for a set of male pregnancies based on one particular informative polymorphic site, as calculated using an embodiment of the current invention (X-axis) versus based on the read counts of chromosome X or Y (Y-axis).

**Figure 30** shows a plot visualizing the estimated fetal fraction for a set of male pregnancies based on the informative polymorphic site identified in the sample as calculated using an embodiment of the current invention (X-axis) versus the estimated fetal fraction based on the read counts of chromosome X or Y (Y-axis).

**DEFINITIONS**

The term "biological sample" as used herein refers to any sample that is taken from a subject (e.g., a human, such as a pregnant woman) and contains one or more nucleic acid molecule(s) of interest.

The term "nucleic acid" or "polynucleotide" refers to a deoxyribonucleic acid (DNA) or ribonucleic acid (RNA) and a polymer thereof in either single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, Single Nucleotide Polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with gene, DNA, cDNA, mRNA, small noncoding RNA, micro RNA (miRNA), Piwi-interacting RNA, and short hairpin RNA (shRNA) encoded by a gene or locus.

The term "gene" means the segment of DNA involved in producing a polypeptide chain. It may include regions preceding and following the coding region (leader and trailer) as well as intervening sequences (introns) between individual coding segments (exons).

The term "reaction" as used herein refers to any process involving a chemical, enzymatic, or physical action that is indicative of the presence or absence of a particular polynucleotide sequence of interest. An example of a "reaction" is an amplification reaction such as a polymerase chain reaction (PCR). Another example of a "reaction" is a sequencing reaction, either by synthesis, ligation, hybridization or by passing the DNA through a pore and measuring signals that are indicative for a particular nucleotide. An "informative reaction" is one that indicates the presence of one or more particular polynucleotide sequence of interest, and in one case where only one sequence of interest is present. The term "well" as used herein refers to a reaction at a predetermined location within a confined structure, e.g., a well-shaped vial, cell, or chamber in a PCR array or e.g. the individual reaction volumes in which sequencing reactions take place (thereby including so-called patterned flow cells from Illumina).

The term "clinically relevant nucleic acid sequence" or "target chromosome or chromosomal segment" as used herein can refer to a polynucleotide sequence corresponding to a segment of a larger genomic sequence whose potential imbalance is being tested or to the larger genomic sequence itself. One example is the sequence of chromosome 21. Other examples include chromosome 18, 13, X and Y. Yet other examples include mutated genetic sequences or genetic polymorphisms or copy number variations (CNVs) that a fetus may inherit from one or both of its parents. Yet other examples include sequences which are mutated, deleted, or amplified in a malignant tumor, e.g. sequences in which loss of heterozygosity or gene duplication occur. In some embodiments, multiple clinically relevant nucleic acid sequences, or equivalently multiple makers of the clinically relevant nucleic acid sequence, can be used to provide data for detecting the imbalance. For instance, data from five non-consecutive sequences on chromosome 21 can be used in an additive fashion for the determination of possible chromosomal 21 imbalance, effectively reducing the need of sample volume to 1/5.

The term "overrepresented nucleic acid sequence" as used herein refers to the nucleic acid sequence among two sequences of interest (e.g., a clinically relevant sequence and a background sequence) that is in more abundance than the other sequence in a biological sample.

The term "based on" as used herein means "based at least in part on" and refers to one value (or result) being used in the determination of another value, such as occurs in the relationship of an input of a method and the output of that method. The term "derive" as used herein also refers to the relationship of an input of a method and the output of that method, such as occurs when the derivation is the calculation of a formula.

The term "parameter" herein refers to a numerical value that characterizes a quantitative data set and/or a numerical relationship between quantitative data sets.

The term "score" as used herein refers to a numerical value which is linked or based on a specific feature, e.g. the number of reads or read counts of a certain sequence present in a sample. The term "first score" is used herein to refer to a numerical value linked to the target chromosome or chromosomal segment. Another example of a score is e.g. a Z score that quantifies how much the number of reads of a certain sequence differs from the number of reads that were obtained from the same sequence in a set of reference samples. It is known to a person skilled in the art how such a Z score can be calculated.

The term "cutoff value" or "threshold" as used herein means a numerical value whose value is used to arbitrate between two or more states (e.g. diseased and non-diseased) of classification for a biological sample. For example, if a parameter is greater than the cutoff value, a first classification of the quantitative data is made (e.g. diseased state); or if the parameter is less than the cutoff value, a different classification of the quantitative data is made (e.g. non-diseased state).

The term "imbalance" as used herein means any significant deviation as defined by at least one cutoff value in a quantity of the clinically relevant nucleic acid sequence from a reference quantity. For example, the reference quantity could be a ratio of 3/5, and thus an imbalance would occur if the measured ratio is 1:1.

The term "random sequencing" as used herein refers to sequencing whereby the nucleic acid fragments sequenced have not been specifically identified or targeted before the sequencing procedure. Sequence-specific primers to target specific gene loci are not required. The pools of nucleic acids sequenced vary from sample to sample and even from analysis to analysis for the same sample. The identities of the sequenced nucleic acids are only revealed from the sequencing output generated. In some embodiments of the present invention, the random sequencing may be preceded by procedures to enrich a biological sample with particular populations of nucleic acid molecules sharing certain common features. In one embodiment, each of the DNA fragments in the biological sample have an equal probability of being sequenced.

The term "fraction of the human genome" or "portion of the human genome" as used herein refers to less than 100% of the nucleotide sequences in the human genome which comprises of some 3 billion basepairs of nucleotides. In the context of sequencing, it refers to less than 1-fold coverage of the nucleotide sequences in the human genome. The term may be expressed as a percentage or absolute number of nucleotides/basepairs. As an example of use, the term may be used to refer to the actual amount of sequencing performed. Embodiments may determine the required minimal value for the sequenced fraction of the human genome to obtain an accurate diagnosis. As another example of use, the term may refer to the amount of sequenced data used for deriving a parameter or amount for disease classification.

The term "summary statistics" as used herein is to be understood as a statistical term, and is to be understood as an indication of the extend of a distribution of values or scores, or as in indication of the score/value present in the middle of the distribution. This can be e.g. a mean or median or standard deviation (StDev) or median absolute deviation (mad) or mean absolute deviation of a collection of scores.

The term "fetal fraction" as used herein refers to the fraction of fetal nucleic acids present in a sample comprising fetal and maternal nucleic acid.

The term "copy number variation" or "CNV" herein refers to variation in the number of copies of a nucleic acid sequence that is a few bp or larger present in a test sample in comparison with the copy number of the nucleic acid sequence present in a qualified sample. A "copy number variant" refers to the few bp or larger sequence of nucleic acid in which copy-number differences are found by comparison of a sequence of interest in test sample with that present in a qualified sample. Copy number variants/variations include deletions, including microdeletions, insertions, including microinsertions, duplications, multiplications. CNVs encompass chromosomal aneuploidies and partial aneuplodies.

The term "aneuploidy" herein refers to an imbalance of genetic material caused by a loss or gain of a whole chromosome, or part of a chromosome. Aneuploidy refers to both chromosomal as well as subchromosomal imbalances, such as, but not limiting to deletions, microdeletions, insertions, microinsertions, copy number variations, duplications. Copy number variations may vary in size in the range of a few bp to multiple Mb, or in particular cases from 1 kb to multiple Mb. Large subchromosomal abnormalities that span a region of tens of MBs and/or correspond to a significant portion of a chromosome arm, can also be referred to as segmental aneuploidies.

The term "chromosomal aneuploidy" herein refers to an imbalance of genetic material caused by a loss or gain of a whole chromosome, and includes germline aneuploidy and mosaic aneuploidy.

The term "partial aneuploidy" herein refers to an imbalance of genetic material caused by a loss or gain of a part of a chromosome e.g. partial monosomy and partial trisomy, and encompasses imbalances resulting from translocations, deletions and insertions.

The terms "polymorphism, polymorphic target nucleic acid", "polymorphic sequence", "polymorphic target nucleic acid sequence" and "polymorphic nucleic acid" are used interchangeably herein to refer to a nucleic acid sequence that contains one or more polymorphic sites.

The term "polymorphic site" herein refers to a single nucleotide polymorphism (SNP), a small-scale multi-base deletion or insertion, a Multi-Nucleotide Polymorphism (MNP) or a Short Tandem Repeat (STR) or a CNV (copy number variation).

The term "plurality" is used herein in reference to a number of nucleic acid molecules or sequence tags or reads that is sufficient to identify significant differences in copy number variations (e.g. chromosome doses) in test samples and qualified samples using the methods of the invention. In some embodiments, at least about $3 \times 10E6$ sequence tags, at least about $5 \times 10E6$ sequence tags, at least about $8 \times 10E6$ sequence tags, at least about $10 \times 10E6$ sequence tags, at least about $15 \times 10E6$ sequence tags, at least about $20 \times 10E6$ sequence tags, at least about $30 \times 10E6$ sequence tags, at least about $40 \times 10E6$ sequence tags, or at least about $50 \times 10E6$ sequence tags are obtained for each test sample. Each sequence tag can be a single sequence read of 20 to 400 bp, or a couple of 2 paired-end sequence reads of each 20 to 400 bp.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA and DNA molecules. The term "polynucleotide" includes, without limitation, single- and double-stranded polynucleotide.

The term "portion" when used in reference to the amount of sequence information of fetal and maternal nucleic acid molecules in a biological sample herein refers to the amount of sequence information of fetal and maternal nucleic acid molecules in a biological sample that in sum amount to less than the sequence information of <1 human genome.

The term "test sample" herein refers to a sample comprising a mixture of nucleic acids comprising at least one nucleic acid sequence whose copy number is suspected of having undergone variation or at least one nucleic acid sequence for which it is desired to determine whether a copy number variation exists. Nucleic acids present in a test sample are referred to as test nucleic acids or target nucleic acids or target chromosomes or target chromosomal segments.

The term "reference sample" herein refers to a sample comprising a mixture of nucleic acids from which the sequencing data are used along with the test sample sequencing data to calculate scores and parameters as described in claim 1. Though not necessary, a reference sample is preferably normal (i.e. not aneuploid) for the sequence of interest. So preferably, a reference sample is a qualified sample that does not carry a trisomy 21 and that can be used for identifying the presence of a trisomy 21 in a test sample.

The term "reference set" comprises a plurality of "reference samples".

The term "enrich" herein refers to the process of specifically amplifying certain target nucleic acids contained in a portion of a maternal sample. The amplified product is than often combined with the remainder of the maternal sample from which the portion was removed.

The term "sequence of interest" herein refers to a nucleic acid sequence that is associated with a difference in sequence representation in healthy versus diseased individuals. A sequence of interest can be a sequence on a chromosome that is misrepresented i.e. over- or under-represented, in a disease or genetic condition. A sequence of interest may also be a portion of a chromosome, or a chromosome. For example, a sequence of interest can be a chromosome that is over-represented in an aneuploidy condition. Sequences of interest include sequences that are over- or under-represented in the total population, or a subpopulation of cells of a subject

The term "group of chromosomes" herein refers to two or more chromosomes. The term "collection" refers to a set of chromosomes or chromosomal segments, but may also refer to a set of values or scores derived from a corresponding set of chromosomes or chromosomal segments.

The term "read" refers to an experimentally obtained DNA sequence of sufficient length (e.g., at least about 20 bp) that can be used to identify a larger sequence or region, e.g. that can be aligned and specifically assigned to a chromosome location or genomic region or gene. The terms 'read' and 'sequences' may be sued interchangeably throughout the draft.

The term "read count" refers to the number of reads retrieved from a sample that are mapped to a reference genome or a portion of said reference genome (bin).

The term "bin" of a genome is to be understood as a segment of the genome. A genome can be divided in several bins, either of a fixed or predetermined size or a variable size. A possible fixed bin size can be e.g. 10 kB, 20 kB, 30 kB, 40 kB, 50 kB, 60 kB, 70 kB, etc. in which kB stands for kilobasepairs, a unit that corresponds to 1000 basepairs.

The term "window" is to be understood as a plurality of bins.

The terms "aligned", "alignment", "mapped" or "aligning", "mapping" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysts pipeline. The matching of a sequence read in aligning can be a 100% sequence match or less than 100% (non-perfect match).

The term "reference genome" as used herein refers to a digital nucleic acid sequence database, assembled as a representative example of a species' DNA. As it is assembled from the sequencing of DNA from multiple, a reference genome does not accurately represent the DNA of a single person. It is used to enable the mapping of sequencing reads from a sample to specific chromosomal positions.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids e.g. cell-free DNA, were naturally released by cells through naturally occurring processes such as necrosis or apoptosis, or through lysis of the cells due to improper storage or transport conditions

The term "maternal sample" herein refers to a biological sample obtained from a pregnant subject e.g. a woman.

The term "biological fluid" herein refers to a liquid taken from a biological source and includes, for example, blood, serum, plasma, sputum, lavage fluid, cerebrospinal fluid, urine, semen, sweat, tears, saliva, blastocoel fluid and the like. It also refers to the medium in which biological samples can be grown, like in vitro culture medium in which cells, tissue or embryo can be cultured. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively. As explained before, "fetal nucleic acids" and "placental nucleic acids" are often used to refer to the same type of nucleic acids, though biological differences may exist between the two types of nucleic acids.

The term "corresponding to" herein refers to a nucleic acid sequence e.g. a gene or a chromosome, that is present in the genome of different subjects, and which does not necessarily have the same sequence in all genomes, but serves to provide the identity rather than the genetic information of a sequence of interest e.g. a gene or chromosome.

The term "substantially cell free" herein refers to preparations of the desired sample from which components that are normally associated with it are removed. For example, a plasma sample is rendered essentially cell free by removing blood cells e.g. white blood cells, which are normally associated with it. In some embodiments, substantially free samples are processed to remove cells that would otherwise contribute to the genetic material that is to be tested for an aneuploidy.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The term "chromosomal segments" is to be understood as a part of a chromosome. Said segment may refer to a bin, window or specific region within a chromosome, e.g. known to comprise for instance deletions or insertions or copy number variations.

As used herein, the term "polynucleotide length" refers to the absolute number of nucleic acid molecules (nucleotides) in a sequence or in a region of a reference genome. The term "chromosome length" refers to the known length of the chromosome given in base pairs.

The term "subject" herein refers to a human subject as well as a non-human subject such as a mammal, an invertebrate, a vertebrate, a fungus, a yeast, a bacteria, and a virus. Although the examples herein concern human genomes and the language is primarily directed to human concerns, the concept of this invention is applicable to genomes from any plant or animal, and is useful in the fields of veterinary medicine, animal sciences, research laboratories and such.

The term "condition" herein refers to "medical condition" as a broad term that includes all diseases and disorders, but can include injuries and normal health situations, such as pregnancy, that might affect a person's health, benefit from medical assistance, or have implications for medical treatments.

The term "attribute" is to be understood as a property or value of an object or element. This can e.g. be a certain correction factor that is used to correct the read count for a particular polymorphism. An attribute may have been experimentally defined using a set of samples.

## DETAILED DESCRIPTION

The invention pertains to a method for determining the presence or absence of a fetal chromosomal aneuploidy in a pregnant female. This determination may be done by the calculation of a parameter linked to chromosomal data obtained from a biological sample. Also provided is a computer readable medium encoded with a plurality of instructions for controlling a computer system to perform the methods.

In a second aspect, the current invention describes a methodology for the determination of the fetal fraction in a sample. In particular, the method enables the determination of the fraction of cell-free DNA (cfDNA) contributed by a fetus to the mixture of fetal and maternal cfDNA in a maternal sample e.g. a plasma sample. In a preferred embodiment, the current invention allows both the determination of the presence or absence of a fetal chromosomal aneuploidy in a pregnant female and the determination of the fetal fraction, independent from the gender of the fetus.

In one aspect, read counts are determined from the sequencing of nucleic acid molecules in a maternal sample, such as urine, plasma, serum, blastocoel fluid and other suitable biological samples. Nucleic acid molecules of the biological sample are randomly sequenced, such that a fraction of the genome is sequenced. One or more cutoff values are chosen for determining whether a change compared to a reference quantity exists (i.e. an imbalance), for example, with regards to the ratio of amounts of two chromosomal regions (or sets of regions).

The change detected in the reference quantity may be any deviation (upwards or downwards) in the relation of the clinically-relevant nucleic acid sequence or target chromosome or chromosomal segment to the other non-clinically-relevant sequences. Thus, the reference state may be any ratio or other quantity (e.g. other than a 1-1 correspondence), and a measured state signifying a change may be any ratio or other quantity that differs from the reference quantity as determined by the one or more cutoff values.

The clinically relevant chromosomal region (also called a clinically relevant nucleic acid sequence or target chromosome or chromosomal segment) and the background nucleic acid sequence may come from a first type of cells and from one or more second types of cells. For example, fetal nucleic acid sequences originating from fetal/placental cells are present in a biological sample, such as maternal plasma, which contains a background of maternal nucleic acid sequences originating from maternal cells. Note the percentage of fetal sequences in a sample may be determined by any fetal-derived loci and not limited to measuring the clinically-relevant nucleic acid sequences.

I. General method for evaluating an aneuploidy

The current invention describes a methodology for detecting the presence or absence of a fetal chromosomal aneuploidy and/or the determination of the fetal fraction present in a biological sample.

In a first aspect, the method for detecting the presence or absence of a fetal chromosomal aneuploidy is based on the determination of a parameter from the nucleic acid content of a biological sample. The biological sample may be plasma, urine, serum, blastocoel fluid or any other suitable sample. The sample contains nucleic acid molecules from the fetus and the pregnant female. For example, the nucleic acid molecules may be fragments from chromosomes.

At least a portion of a plurality of the nucleic acid molecules contained in the biological sample is randomly sequenced to obtain a number of sequences. The portion sequenced represents a fraction of the human genome and may be isolated from the sample by conventional means (e.g. cell-free DNA extraction and preparation of a NGS library). In one embodiment, the nucleic acid molecules are fragments of respective chromosomes. One end (e.g. 50 basepairs (bp)), both ends, or the entire fragment may be sequenced. A subset of the nucleic acid molecules in the sample may be sequenced, and this subset is randomly chosen, as will be described in more detail later.

In one embodiment, the random sequencing is done using massively parallel sequencing. Massively parallel sequencing, such as that achievable on the HiSeq2000, HiSeq2500, HiSeq3000, HiSeq4000, HiSeq X, MiSeq, MiSeqDx, NextSeq500, NextSeq550 flowcell, the 454 platform (Roche), Illumina Genome Analyzer (or Solexa platform) or SOLiD System (Applied Biosystems) or PGM or Proton platform (IonTorrent) or GeneRead (Qiagen) or the Helicos True Single Molecule DNA sequencing technology, the single molecule, real-time (SMRT™) technology of Pacific Biosciences, and nanopore sequencing as in MinION, PromethION, GridION (Oxford Nanopore technologies), allow the sequencing of many nucleic acid molecules isolated from a specimen at high orders of multiplexing in a parallel fashion. Each of these platforms sequences clonally expanded or even non-amplified single molecules of nucleic acid fragments. Clonal expansion can be achieved via bridge amplification, emulsion PCR, or Wildfire technology.

As a high number of sequencing reads, in the order of hundred thousand to millions or even possibly hundreds of millions or billions, are generated from each sample in each run, the resultant sequenced reads form a representative profile of the mix of nucleic acid species in the original specimen. For example, the haplotype, transcriptome and methylation profiles of the sequenced reads resemble those of the original specimen. Due to the large sampling of sequences from each specimen, the number of identical sequences, such as that generated from the sequencing of a nucleic acid pool at several folds of coverage or high redundancy, is also a good quantitative representation of the count of a particular nucleic acid species or locus in the original sample.

Based on the sequencing (e.g. data from the sequencing), a first score of a target chromosome or chromosomal segment (e.g. the clinically relevant chromosome) is determined. The first score is determined from sequences identified as originating from (i.e. aligning) to the target chromosome or chromosomal segment. For example, a bioinformatics procedure may then be used to locate each of these DNA sequences to the human genome or a reference genome. It is possible that a proportion of such sequences will be discarded from subsequent analysis because they are present in the repeat regions of the human genome, or in regions subjected to inter-individual variations, e.g. copy number variations. A score of the target chromosome or chromosomal segment and of one or more other chromosomes may thus be determined.

Based on the sequencing, a collection of scores of one or more chromosomes or chromosomal segments is determined from sequences identified as originating from (i.e. aligning to) a set of one of more chromosomes. In one embodiment, said set contains all of the other chromosomes besides the first one (i.e. the one being tested). In another embodiment, said set contains just a single other chromosome. In a most preferred embodiment, said set contains chromosomes or chromosomal segments and includes the target chromosome or chromosomal segment.

There are a number of ways of determining a score. By preference, said score is based on the read counts obtained from sequencing. Said read counts can include, but are not limiting to counting the number of sequenced reads, the number of sequenced nucleotides (basepairs) or the accumulated lengths of sequenced nucleotides (basepairs) originating from particular chromosome(s) or chromosomal segments such as bins or windows or clinically-relevant chromosome portions.

Rules may be imposed on the results of the sequencing to determine what gets counted. In one aspect, a read count may be obtained based on a proportion of the sequenced output. For example, sequencing output corresponding to nucleic acid fragments of a specified size range could be selected.

In one embodiment, said score is the raw read count for a certain chromosome or chromosomal segment.

In a preferred embodiment, said read counts are subjected to mathematical functions or operations in order to derive said score of said read counts. Such operations include but are not limiting to statistical operations, regression models standard calculations (sum, subtraction, multiplying and division), whereby said standard calculations are preferably based on one or more obtained read counts.

In a preferred embodiment, said first score is a normalized value derived from the read counts or mathematically modified read counts. In a further preferred embodiment, said score is a Z score or standard score relating to the read counts of a certain chromosome, chromosomal segment, or the mathematically amended counts thereof, in which the Z score quantifies how much the number of reads of a certain sequence differs from the number of reads that were obtained from the same sequence in a set of reference samples. It is known to a person skilled in the art how such a Z score can be calculated.

In a preferred embodiment, a parameter is determined based on a first score (corresponding to the target chromosome or chromosomal segment) and a collection of scores. The parameter preferably represents a relative score between the first score and a summary statistic of the collection of scores. The parameter may be, for example, a simple ratio of the first score to a summary statistic of the collection of scores. In one aspect, each score could be an argument to a function or separate functions, where a ratio may be then taken of these separate functions.

In a preferred embodiment, the parameter may be obtained by a ratio between:

- a first function whereby the first score and the collection of scores are the arguments;
- a second function whereby the collection of scores is the argument.

In a more preferred embodiment, said first function is defined as a difference, preferably the difference between the first score and a summary statistic of the collection of scores, whereby said summary statistic is preferably selected from the mean, median, standard deviation or median absolute deviation (mad) or mean absolute deviation.

In a further preferred embodiment, said second function is defined as a variability summary statistic of the collection of scores, whereby said summary statistic may be an average or a measure of variability and is preferably selected from the mean, median, standard deviation or median absolute deviation (mad) or mean absolute deviation.

Typically, a suitable embodiment according to the current invention involves the following steps (after having obtained DNA sequences from a random, low-coverage sequencing process on a biological sample).

- aligning sequences to a reference genome;
- obtaining the read counts per chromosome or chromosomal segment;
- normalizing the number of reads or a derivative thereof towards a normalized number of reads;
- obtaining a first score derived from said normalized number of reads and a collection of scores derived from said normalized reads, whereby said first score is derived from the normalized read counts for a target chromosome or chromosomal segment , and said collection of scores is a set of scores derived from the normalized number of reads that were obtained from a set of chromosomes or chromosome segments that include the target chromosomal segment or chromosome;
- calculating a parameter from said scores, whereby said parameter represents a ratio between said first score and a summary statistic of said collection of scores, whereby the first function of said ratio is defined as a difference between the first score and a summary statistic of said collection of scores; and whereby the second function of said ratio is defined as a summary statistic of said collection of scores.

Preferably, said sequences are obtained by low coverage sequencing.

Said normalization preferably occurs on the basis of a set of reference samples, whereby said reference samples are preferably, though not necessary, euploid or essentially euploid for the chromosome or chromosomal segment that corresponds to the target chromosome or chromosomal segment (i.e. the majority of the chromosomes or chromosomal segments in the reference samples that correspond to the target chromosome or chromosomal segment in the test sample are euploid). Such reference set have various sample sizes. A possible sample size can be e.g. 100 samples, such as 50 male and 50 female samples. It will be understood by a skilled person that the reference set can be freely chosen by the user.

By preference, said number of reads is recalibrated to correct for GC content and/or total number of reads obtained from said sample.

By taking into account a set of scores derived of reads of chromosomes or chromosomal segments that include the target chromosome or chromosomal segment, a more robust, sensitive and reliable parameter is obtained as compared to known prior art methods. Other than the known prior art methods, there is no need to make an assumption on the ploidy state of any of the chromosomes in the test sample. In fact, by defining a parameter according to the current invention, the parameter for the chromosome or region to be analyzed clearly stands out (i.e. is strongly increased/decreased), and does not disappear in the noise (i.e. only moderately or not increased/decreased). Moreover, for screening purpose, sensitivity is key, as it is important to have a reliable and trustworthy result, thereby minimizing the amount of false negatives. In fact, for screening purposes, it may be more important to have high superiority as compared to specificity.

The parameter according to the current invention allows robustly detecting and automatically classifying chromosomes, even in noisy data. By taking into account a collection of chromosomes or segments, including the target chromosome or segment, i.e. the majority of information that is available in the dataset, most of the available information is used, coming to a more adequate assessment. For instance, if one would remove e.g. chromosome 1 (the largest chromosome, 7.9% of the genome), a large amount of data would be removed that would not be taken into account, thereby causing a distortion in the assessment.

In particular, the current invention is very useful in situations whereby a low number of reads or noisy data is obtained. The inventors found that in the latter situations, the parameter according to the current invention performed superior compared to other methodologies.

In a preferred embodiment, said scores are obtained on the basis of the genomic representation of the target chromosome or chromosomal segment (or a region thereof) and the genomic representation of all autosomes or chromosomes, thereby including the target chromosome or chromosomal segment.

The parameter is compared to one or more cutoff values. The cutoff values may be determined from any number of suitable ways. Such ways include Bayesian-type likelihood method, sequential probability ratio testing (SPRT), false discovery, confidence interval, receiver operating characteristic (ROC). In a more preferred embodiment, said cutoff value is based on statistical considerations or is empirically determined by testing biological samples. The cutoff value can be validated by means of test data or a validation set and can, if necessary, be amended whenever more data is available.

It is possible that in some variants of the procedure, the cutoff value would be adjusted in accordance with information on the fraction of the of cell-free fetal DNA in the maternal plasma sample (also termed fetal fraction or abbreviated as ff or f). In another embodiment, said fetal fraction may serve as an internal control of the quality of the sample. The value of f can be determined from the sequencing dataset in different ways (dependent on the gender of the fetus, or independent from the gender of the fetus), as will be explained further on.

Based on the comparison, a classification of whether a fetal chromosomal aneuploidy exists for the target chromosome or chromosomal section is determined. In one embodiment, the classification is a definitive yes or no. In another embodiment, a classification may be unclassifiable or uncertain. In yet another embodiment, the classification may be a risk score that is to be interpreted at a later date, for example, by a doctor.

In a further preferred method, secondary parameters from the read counts are calculated, which serve as an additional internal control for the usefulness of the parameter, the extend of the aneuploidy (if identified) and/or an indication for the reliability of the parameter, the biological sample or the sequences obtained thereof and thus the final assessment. The value for said secondary parameters can be e.g. a measure or prerequisite of the presence of said aneuploidy and/or a measure of quality of the sample.

In one embodiment, such secondary parameter is calculated as the median of the Z-distribution of the read counts or a derivative thereof, for a target chromosome or a target chromosomal segment measured per bin or an aggregation of bins (i.e. windows). The latter secondary parameters allow assessing if the majority (more than 50%) of the windows in a chromosome is increased or decreased. The latter allows the detection of chromosomal and large subchromosomal aneuploidies. When less than 50% of the windows are affected, the secondary parameters will not be affected (e.g. for smaller CNVs).

In another embodiment, said secondary parameters may be calculated as the median of the absolute value of the Z-scores for the read counts or a derivative thereof, of the remaining chromosomes (that is a collection of chromosomes or segments that exclude the target chromosome or chromosomal segment).

The latter secondary parameters allow the detection of a.o. the presence of technical or biological instabilities and to discriminate these from maternal CNVs. If less than the windows of the other or all chromosomes are affected, this secondary parameter will not be affected. If more than 50% of the windows is affected, this will be derivable from said secondary parameters.

In another embodiment, the current invention also provides for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and test a new biological sample. This is typical for moderately increased QS scores. A strongly increased QS could be an indication of a highly aneuploid sample and the user will be encouraged to do a confirmatory test. Preferably, said QS is determined by calculating the standard deviations of all Z scores for chromosomes or chromosomal segments and optionally by removing the outliers thereof (i.e. the highest and lowest Z scores in this collection).

As an alternative or additional embodiment, the determination of the fetal fraction (see below) also serves as an internal quality control of the sample and the sequences obtained thereof. The quality of a sample may be hampered after retrieval, e.g. by inappropriate conditions during collection, transport or storage. The latter may have an effect on the cell free DNA in the sample, for instance due to rupture of (maternal) white blood cells. As such, the main pool of free floating DNA will become even more enriched for maternal DNA, thereby reducing the percentage of the fetal fraction compared to the total cell free DNA content in the sample. By preference, said fetal fraction will be determined by at least one of the methods described below.

In an embodiment of the current invention, the parameter will be sufficient to discriminate between the presence and/or absence of an aneuploidy. In a more preferred embodiment of the current invention; both the parameter as the secondary parameters will be used to come to a decision with regard to the presence or absence of an aneuploidy. Preferably, also said secondary parameters will be compared to predefined threshold values.

By preference, the methodology according to the current invention is particularly suitable for analyzing aneuploidies linked to segments or deletions given in Table 1, which contains a not-limiting list of chromosome abnormalities that can be potentially identified by methods and kits described herein. In a another or further embodiment, said target chromosomal segment is selected from a bin or a window derived from chromosome X, Y, 6, 7, 8, 13, 14, 15, 16, 18, 21 and/or 22.

In a further or other embodiment, said target chromosome is selected from chromosome X, Y, 6, 7, 8, 13, 14, 15, 16, 18, 21 and/or 22.

**Table 1**

| Chromosome | Abnormality | Disease Association |
|---|---|---|
| X | XO | Turner's Syndrome |
| Y | XXY | Klinefelter syndrome |
| | XYY | Double Y syndrome |
| | XXX | Trisomy X syndrome |
| | XXXX | Four X syndrome |
| | Xp21 deletion | Duchenne's/Becker syndrome, congenital adrenal hypoplasia, chronic granulomatous disease |
| | Xp22 deletion | Steroid sulfatase deficiency |
| | Xp26 deletion | X-linked lymph proliferative disease |
| 1 | 1p Monosomy, trisomy | |
| | 1p36 | 1p36 deletion syndrome |
| | 1q21.1 | 121.1 deletion syndrome; distal 1q21 deletion sydnrome |
| 2 | Monosomy, trisomy 2q | Growth retardation, developmental and mental delay, and minor physical abnormalities |
| | 2p15-16.1 | 2p15-16.1 deletion syndrome |
| | 2q23.1 | 2q23.1 deletion syndrome |
| | 2q37 | 2q37 deletion syndrome |
| 3 | Monosomy, trisomy | |
| | 3p | 3p deletion syndrome |
| | 3q29 | 3q29 deletion syndrome |
| 4 | Monosomy, trisomy | |
| | 4p- | Wolf-Hirschhorn syndrome |
| 5 | 5p | Cri du chat; Lejeune syndrome |
| | 5q Monosomy, trisomy | Myelodysplastic syndrome |
| | 5q35 | 5q35 deletion syndrome |
| 6 | Monosomy, trisomy | |
| | 6p25 | 6p25 deletion syndrome |
| 7 | 7q11.23 deletion | William's syndrome |
| | Monosomy, trisomy | Monosomy 7 syndrome of childhood; myelodysplastic syndrome |
| 8 | 8q24.1 deletion | Langer-Giedion syndrome |
| | 8q22.1 | Nablus mask-like facial syndrome |
| | Monosomy, trisomy | Myelodysplastic syndrome; Warkany syndrome; |
| 9 | Monosomy 9p | Alfi's syndrome |
| | Monosomy 9p, partial | Rethore syndrome |
| | trisomy 9p | |
| | trisomy | Complete trisomy 9 syndrome; mosaic trisomy 9 syndrome |
| | 9p22 | 9p22 deletion syndrome |

(continued)

| Chromosome | Abnormality | Disease Association |
|---|---|---|
|  | 9q34.3 | 9q34.3 deletion syndrome |
| 10 | Monosomy, trisomy | ALL or ANLL |
|  | 10p14-p13 | DiGeorge's syndrome type II |
| 11 | 11p- | Aniridia; Wilms tumor |
|  | 11p13 | Wagr syndrome |
|  | 11p11.2 | Potocki Shaffer syndrome |
|  | 11p15 | Beckwith-Wiedemann syndrome |
|  | 11q- | Jacobsen syndrome |
|  | Monosomy, trisomy |  |
| 12 | Monosomy, trisomy |  |
| 13 | 13q- | 13q-syndrome; Orbeli syndrome |
|  | 13q14 deletion |  |
|  | Monosomy, trisomy | Patau's syndrome |
| 14 | Monosomy, trisomy |  |
| 15 | 15q11-q13 deletion, monosomy | Prader-Willi, Angelman's syndrome |
|  | Trisomy |  |
| 16 | 16q13.3 deletion | Rubenstein-Taybi |
|  | Monosomy, trisomy |  |
| 17 | 17p- | 17p syndrome |
|  | 17q11.2 deletion | Smith-Magenis |
|  | 17q13.3 | Miller-Dieker |
|  | Monosomy, trisomy |  |
|  | 17p11.2-12 trisomy | Charcot-Marie Tooth Syndrome type 1; HNPP |
| 18 | 18p- | 18p partial monosomy syndrome or Grouchy Lamy Thieffry syndrome |
|  | Monosomy, trisomy | Edwards Syndrome |
| 19 | Monosomy, trisomy |  |
| 20 | 20p- | Trisomy 20p syndrome |
|  | 20p11.2-12 deletion | Alagille |
|  | 20q- |  |
|  | Monosomy, trisomy |  |
| 21 | Monosomy, trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
|  | Monosomy, trisomy | Complete trisomy 22 syndrome |

II Sequencing, aligning and correction

As mentioned above, only a fraction of the genome is sequenced. In one aspect, even when a pool of nucleic acids in a specimen is sequenced at <100% genomic coverage instead of at several folds of coverage, and among the proportion of sequenced nucleic acid molecules, most of each nucleic acid species is not sequenced or sequenced only once.

This is contrasted from situations where targeted enrichment is performed of a subset of the genome prior to the sequencing reaction, followed by high-coverage sequencing of that subset.

In one embodiment, said sequences are obtained by next generation sequencing. In a further preferred embodiment, said sequencing method is a low coverage, random sequencing method.

In one embodiment, massive parallel short-read sequencing is used. Short sequence tags or reads are generated, e.g. from a certain length between 20bp and 400bp. Alternatively, paired end sequencing could be performed.

In an embodiment, a pre-processing step is available for pre-processing the obtained reads. Such pre-processing option allows filtering low quality reads, thereby preventing them from being mapped. Mapping of low quality reads can require prolonged computer processing capacity, can be incorrect and risks increasing the technical noise in the data, thereby obtaining a less accurate parameter. Such pre-processing is especially valuable when using next-generation sequencing data, which has an overall lower quality or any other circumstance which is linked to an overall lower quality of the reads.

The generated reads can subsequently be aligned to one or more human reference genome sequences. Preferably, the number of aligned reads are counted and/or sorted according to their chromosomal location.

An additional clean-up protocol can be performed, whereby deduplication is performed, e.g. with Picard tools, retaining only uniquely mapped reads. Reads with mismatches and gaps can be removed. Reads that map to blacklisted regions can be excluded. Such blacklisted regions may be taken from a predefined list of e.g. common CNVs, collapsed repeats, DAC blacklisted regions as identified in the ENCODE project (i.e. a set of regions in the human genome that have anomalous, unstructured, high signal/read counts in NGS experiments independent of cell line and type of experiment) and the undefined portion of the reference genome. In one embodiment, blacklisted regions are provided to the user. In another embodiment, the user may use or define his or her own set of blacklisted regions.

In a further embodiment, chromosomes are divided into regions of a predefined length, generally referred to as bins. In an embodiment, the bin size is a predefined size provided to the user. In another embodiment, said bin size can be defined by a user, can be uniformly for all chromosomes, can be a specific bin size per chromosome or can vary according to the obtained sequence data. Change of the bin size can have an effect on the final parameter to be defined, either by improving the sensitivity (typically obtained by decreasing the bin size, often at the cost of the specificity) or by improving the specificity (generally by increasing the bin size, often at the cost of the sensitivity). A possible bin size which provides an acceptable specificity and sensitivity is 50 kb.

In a further step, the aligned and filtered reads within a bin are counted, in order to obtain read counts.

The obtained read counts can be corrected for the GC count for the bin. GC bias is known to aggravate genome assembly. Various GC corrections are known in the art (e.g. Benjamini et al., Nucleic Acid Research 2012). In a preferred embodiment, said GC correction will be a LOESS regression. In one embodiment, a user of the methodology according to the current invention can be provided with the choice of various possible GC corrections.

In a subsequent step, the genomic representation (GR) of read counts per bin is calculated. Such representation is preferably defined as a ratio between the GC-corrected read counts for a specific bin and the sum of all GC-corrected read counts.

In an embodiment, said GR is defined as follows:

$$GRi = \frac{GCi}{\sum_k GCk} \cdot 10^7$$

with k over all chromosomal bins

The factor $10^7$ (or 10E7) in the above formula is arbitrary defined, and can be any constant value.

In a final step, the obtained GR per bin are aggregated over a region, whereby said region may be a subregion (window) of a chromosome or the full chromosome. Said window may have a predefined or variable size, which can optionally be chosen by the user. A possible window could have a size of 5 MB or 100 adjacent bins of size 50 kb.

The GR aggregated for a chromosome can be defined by

$$GRi = \frac{\sum_{j \in (bins\ on\ chr)} GCi}{\sum_{k \in (all\ bins) GCk} GCk}$$

In a further embodiment, the genomic representation of a set of reference samples shall be calculated. Said set of reference samples (or also termed reference set) can be predefined or chosen by a user (e.g. selected from his/her own reference samples). By allowing the user the use of an own reference set, a user will be enabled to better capture the recurrent technical variation of his/her environment and its variables (e.g. different wet lab reagents or protocol, different NGS instrument or platform, etc.). In a preferred embodiment, said reference set comprises genomic information of 'healthy' samples that are expected or known to not contain (relevant) aneuploidies. The genomic representation (GR) of the reference set can be defined, either at the genome level and/or at a subregion (chromosome, chromosomal segment, window, or bin).

Other single molecule sequencing strategies such as that by the Roche 454 platform, the Applied Biosystems SOLiD platform, the the Helicos True Single Molecule DNA sequencing technology, the single molecule, real-time (SMRT™) technology of Pacific Biosciences, and nanopore sequencing technologies like MinION, GridION or PromethION from Oxford Nanopore Technologies could similarly be used in this application.

III Determining scores, parameter and secondary parameters

From the alignments and the obtained read counts or a derivative thereof, optionally corrected for GC content and/or total number of reads obtained from said sample, scores are calculated which eventually lead to a parameter allowing the determination of the presence of an aneuploidy in a sample. Said scores are normalized values derived from the read counts or mathematically modified read counts, whereby normalization occurs in view of the reference set. As such, each score is obtained by means of a comparison with the reference set. The term first score is used to refer to score linked to the read count for a target chromosome or a chromosomal segment. A collection of scores is a set of scores derived from a set of normalized number of reads that may include the normalized number of reads of said target chromosomal segment or chromosome.

Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

In a most preferred embodiment, the first score and the collection of scores are calculated on the basis of the genomic representation of either a target chromosome or chromosomal segment, or all autosomes or chromosomes (or regions thereof) thereby including the target chromosome or chromosome segment.

Such scores can be calculated as follows:

$$Zi = \frac{GRi - \mu\,ref,i}{\sigma\,ref,i}$$

With i a window or a chromosome or a chromosome segment.

A summary statistic of said collection of scores can e.g. be calculated as the mean or median value of the individual scores. Another summary statistic of said collection of scores can be calculated as the standard deviation or median absolute deviation or mean absolute deviation of the individual scores.

Said parameter p will be calculated as a function of the first score and a derivative (e.g. summary statistic) of the collection of scores. In a preferred embodiment, said parameter will be a ratio between the first score corrected by the collection of scores (or a derivative thereof) and a derivative of said collection of scores.

In another embodiment, said parameter will be a ratio between the first score corrected by a summary statistic of a first collection of scores and a summary statistic of a different, second collection of scores, in which both collections of scores include the first score.

In a specifically preferred embodiment, said parameter p is a ratio between the first score, corrected by a summary statistic of said collection of scores, and a summary statistic of said collection of scores. Preferably, the summary statistic is selected from the mean, median, standard deviation, median absolute deviation or mean absolute deviation. In one embodiment, said both used summary statistics in the function are the same. In another, more preferred embodiment, said summary statistics of the collection of scores differ in the numerator and denominator.

Typically, a suitable embodiment according to the current invention involves the following steps (after having obtained DNA sequences from a random sequencing process on a biological sample).

- aligning said obtained sequences to a reference genome;
- counting the number of reads on a set of chromosomal segments and/or chromosomes thereby obtaining read counts;
- normalizing said read counts or a derivative thereof into a normalized number of reads;
- obtaining a first score and a collection of scores of said normalized reads, whereby said first score is derived from the normalized reads for a target chromosome or chromosomal segment and said collection of scores is a set of scores derived from a corresponding set of chromosomes or chromosome segments that include said target chromosomal segment or chromosome;

- calculating a parameter p from said first score and said collection of scores, whereby said parameter represents a ratio between
  * said first score, corrected by a summary statistic of said collection of scores, and
  * a summary statistic of said collection of scores.

A possible parameter p can be calculated as follows:

$$Z \, \text{of} \, Z_i = \frac{Z_i - \underset{j=i,a,b,\ldots}{\mathrm{median}}\left(Z_j\right)}{\underset{j=i,a,b,\ldots}{\mathrm{sd}}\left(Z_j\right)}$$

Whereby Zi represents the first score and $Z_j$ the collection of scores and whereby i represents the target chromosome or chromosomal section, and whereby j represents a collection chromosomes or chromosomal segments i, a, b, .. that include said target chromosomal segment or chromosome i.

In another embodiment, said parameter p is calculated as

$$Z \, \text{of} \, Z_i = \frac{Z_i - \underset{j=i,a,b,\ldots}{\mathrm{mean}}\left(Z_j\right)}{\underset{j=i,a,b,\ldots}{\mathrm{mad}}\left(Z_j\right)}$$

Whereby $Z_i$ represents the first score and $Z_j$ the collection of scores and whereby i represents the target chromosome or chromosomal section, and whereby j represents a collection of chromosomes or chromosomal segments i, a, b, .. that includes said target chromosomal segment or chromosome i.

In yet another, most preferred embodiment, said parameter p is calculated as

$$Z \, \text{of} \, Z_i = \frac{Z_i - \underset{j=i,a,b,\ldots}{\mathrm{median}}\left(Z_j\right)}{\underset{j=i,a,b,\ldots}{\mathrm{mad}}\left(Z_j\right)}$$

Whereby Zi represents the first score and $Z_j$ the collection of second scores and whereby i represents the target chromosome or chromosomal section, and whereby j represents a collection of chromosomes or chromosomal segments i, a, b, .. that includes said target chromosomal segment or chromosome i.

Apart from the parameter p which will allow the identification of the presence of an aneuploidy, secondary parameters can be calculated which may serve as quality control or provide additional information with regard to one or more aneuploidies present in the sample.

A first secondary parameter which can be calculated allows defining whether chromosomal and large subchromosomal aneuploidies are present in the sample (compared to e.g. smaller aneuploidies). In a preferred embodiment, such parameter is defined by the median of Z scores measured per subregions (e.g. 5 Mb windows) in a target chromosome or target chromosomal section. If more than 50% of these subregions are affected, this will show in the secondary parameter.

In another embodiment, a secondary parameter may be calculated as the median of the absolute value of the Z scores calculated over the remaining chromosomes (that is all chromosomes except the target chromosome or chromosomal segment) per subregion (e.g. 5 Mb windows).

The latter secondary parameter allows the detection of the presence of technical or biological instabilities. If less than half of the windows of the other or all chromosomes are affected, this secondary parameter will not be affected. If more than 50% of the windows is affected, this will be derivable from said secondary parameter.

In another embodiment, the current invention also provides for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and sequence a new biological sample. This is typical for moderately increased QS scores. A strongly increased QS may be an indication of a highly aneuploid sample and the user will be encouraged to do a confirmatory test. Preferably, said QS is determined by calculating the standard deviations of all Z scores for the autosomes or chromosomes and by removing the highest and lowest scoring chromosome.

For instance, samples with a QS exceeding 2 are considered to be of poor quality , and a QS between 1.5 and 2 are of intermediate quality.

IV. Comparison to cutoff value

The parameter p as calculated in the embodiments above shall subsequently be compared with a cutoff parameter for determining whether a change compared to a reference quantity exists (i.e. an imbalance), for example, with regards to the ratio of amounts of two chromosomal regions (or sets of regions). In one embodiment, the user will be able to define its own cutoff value, either empirically on the basis of experience or previous experiments, or for instance based on standard statistical considerations. If a user would want to increase the sensitivity of the test, the user can lower the thresholds (i.e. bring them closer to 0). If a user would want to increase the specificity of the test, the user can increase the thresholds (i.e. bring them further apart from 0). A user will often need to find a balance between sensitivity and specificity, and this balance is often lab- and application -specific, hence it is convenient if a user can change the threshold values him- or herself.

Based on the comparison with the cutoff value, an aneuploidy may be found present or absent.

In an embodiment of the current invention, comparison of parameter p with a cutoff value is sufficient for determining the presence or absence of an aneuploidy. In another embodiment, said aneuploidy is determined on the basis of a comparison of parameter p with a cutoff value and a comparison of at least one of the secondary parameters, quality score and/or first score with a cutoff value, whereby for each score a corresponding cutoff value is defined or set.

In a preferred embodiment, said presence/absence of an aneuploidy is defined by a comparison of parameter p with a predefined cutoff value, as well as by comparison of all secondary parameters, quality and first scores as described above with their corresponding cutoff values.

The final decision tree may thus be dependent on parameter p alone, or combined with one of the secondary parameters and/or quality score or first score as described above.

In a preferred embodiment, said methodology according to the current invention comprises the following steps:

- multiplex sequencing of 50 bp single-end reads (performed by end user)
- uploading sequence reads
- mapping of reads to a reference genome
- count number of reads per bin (a bin has a size of 50 kb)
- compute GC content per bin and correct for GC content
- compute Genomic Representation (GR) score per bin. For bin i this equals

$$GRi = \frac{\sum_{j \in (bins\ on\ chr)} GCi}{\sum_{k \in (all\ bins)GCk} GCk}$$

whereby GCi represents the GC corrected number of reads for bin i, and GCk represents the GC corrected number of reads for bin k.

- aggregate the GR values per window (a window consists out of 100 consecutive windows)
- compute a Z score per window or per chromosome, whereby the Z score is based on the GR score per chromosome, compared with the GR scores obtained in a set of reference samples.

$$Z_i = \frac{GR_i - \mu_{Ref,i}}{\sigma_{Ref,i}}$$

with i a chromosome or a window, $\mu_{Ref,i}$ the average or median GR score for the corresponding bins in the set of reference samples and $\sigma_{Ref,I}$ the standard deviation of the GR scores for the corresponding bins in the set of reference samples

- computing of a ZofZ score, whereby the ZofZ score is based on the Z score, corrected by the median (or mean) of the Z scores of a collection of chromosomes or chromosome segments including target chromosome i and divided by a factor that measures the variability of the Z scores of a collection of chromosomes that includes the target chromosome i (standard deviation or a more robust version thereof, like e.g. the median absolute deviation or mad).
- comparison of the Z score with a threshold value, and the ZofZ score with a threshold value, to predict the presence or absence of an aneuploidy.

In a further preferred embodiment, said prediction of the presence or absence of an aneuploidy occurs via a decision tree based on parameter p and secondary parameters.

V. Determination of Fetal Fraction

Apart or next to the determination of the presence of an aneuploidy, the current invention also provides for one or more methodologies for determining the fetal fraction or fetal nucleic acids in a sample which is a mixture of fetal and maternal nucleic acids. In general, the fetal fraction within the sample will be so low that it cannot be easily determined. In general, the fetal fraction in samples will vary from 4 to 20% across different samples, with an average of about 10% of the total genome fraction.

The current invention provides for two different methodologies of defining the fetal fraction in a sample, depending on the nature of the pregnancy.

A first methodology is independent of the type of pregnancy or sex of the fetus. Such methodology is based on the presence of polymorphisms in the maternal (and fetal) DNA of the sample. More specifically, it is based on predefine stretches of DNA that can be present in the fetal DNA and absent in the maternal DNA. Polymorphic sites that are contained in the target nucleic acids include without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs), Copy Number Variations (CNVs) and Short Tandem Repeats (STRs). In a most preferred embodiment, said polymorphisms are CNVs. In a first step, the sample is sequenced as described above and reads are mapped against a reference genome.

In a subsequent step, the number of sequences that align to each of a predefined set of polymorphisms is counted. Optionally this can be achieved by mapping the obtained sequence reads to each of said predefined polymorphisms. Said predefined set of polymorphisms is to be understood as a collection of polymorphisms which have been identified and are deemed to be of relevance for the determination of the fetal fraction.

Said set of polymorphisms are preferably benign polymorphisms with frequent occurrence in population. In a preferred embodiment, said set comprises CNVs. Such CNVs may be variable in size, in a preferred embodiment, said CNVs have a length between 10 kb to 1Mb, more preferably between 10 kb and 100 kb. Alternatively, said CNVs have a length between 2 bp and 10 Mb. Herein kb refers to kilobasepairs (i.e. 1,000 basepairs) and Mb refers to megabasepairs (i.e. 1,000,000 basepairs)

In a further embodiment, said set of polymorphisms comprises additional data which is linked to the polymorphisms within said set. In a preferred embodiment, said data comprises one or more attributes of each polymorphism. Said attributes may comprise, but are not limiting to a correction factor for each polymorphism, whereby said correction factor provides a link between read counts for said polymorphism and the actual fetal fraction. The latter allows for a simple correction of the obtained reads corresponding to a polymorphism within a sample thereby obtaining an estimate of the fetal fraction or the actual fetal fraction.

Said attributes may comprises a cutoff value per polymorphism which allows identifying whether said polymorphism, if present in a sample, qualifies as informative polymorphism.

In a preferred embodiment, said attributes are determined using a set of samples for which the fetal fraction is known. These samples could be e.g. male pregnancies (for which the fetal fraction can be determined using the reads coming from the X or Y chromosome, as described further), or alternatively, samples for which the fetal fraction was determined using orthogonal methods (based on epigenetics or targeted sequencing or digital PCR).

In an embodiment, said set of polymorphisms and attributes are predefined. In another embodiment, said set of polymorphisms and attributes may be defined by the user.

In a subsequent step, the obtained read count - or a derivative thereof - for each polymorphism is used to identify whether the particular polymorphism is informative in the sample. In general, such informative polymorphisms are those polymorphisms which have a lower read number than a certain cut-off, in which the cut-off may correspond to the theoretically expected number of reads given the total read number for the sample, or a derivative thereof. It is deemed that the lower amount of observed read counts for such informative polymorphisms is due to their presence in the fetal genome and not in the maternal genome. Preferably, the number of reads - or a derivative thereof - should be less than half of the theoretically expected number of reads, as this would mean that the polymorphism is not present in 1, 2 or more copies in the maternal genome and hence only present in the fetal genome. Based on these informative polymorphisms, an estimation of the fetal fraction can be made by assuming that the obtained read count is directly correlated with the fetal fraction. In a preferred embodiment, the fetal fraction is calculated by first correcting the obtained read count for each informative polymorphism using its polymorphism specific attributes, and subsequently taking the median or average of the corrected read counts across all informative polymorphisms. A sample should have at least one informative polymorphism in order to be able estimate the fetal fraction. The fetal fraction of samples for which no informative polymorphism was identified can be estimated using alternative methods, provided that the sample is derived from a male fetus (see below).

In one embodiment, the fraction of fetal nucleic acids in the mixture of fetal and maternal nucleic acids is calculated for each of the informative polymorphism. In a first step, the expected count for the informative polymorphism is computed based on the normalized counts (normalized towards e.g. 10.000.000) obtained from said sample. Subsequently, based on the expected counts (i.e. the number of reads that one would expect for the polymorphism, given the total number of reads obtained for the sample, and optionally corrected with a polymorphism specific attribute), an estimation of the fetal fraction of the test sample is derived.

In an embodiment, this estimation can be computed for each informative polymorphism, using the formula 2 x 100 x observed counts for the informative polymorphism / expected counts for the informative polymorphism.

The expected count is the number of reads that one would expect for the polymorphism, given the total number of reads obtained for the sample, and optionally corrected with a polymorphism specific attribute. This polymorphism specific attribute or factor can be derived for each polymorphism in said set of polymorphisms using a set of samples for which the fetal fraction is known using alternative methods (e.g. for male fetuses using read counts on chromosome X or Y).

In an embodiment, the actual fetal fraction can be calculated as follows:

$$\text{Actual fetal fraction}= \text{estimated fetal fraction x factor.}$$

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 37, at least 18, at least 19, at least 20, at least 25, at least 30, at least 35, at least 40 or more informative polymorphisms. In an embodiment, the fetal fraction will be determined by the average or median fetal fraction as determined for each individual informative polymorphisms. In one embodiment, the fetal fraction is the average or median fetal fraction determined for at least 1, 2 or 3 informative polymorphisms.

In general, determination of the fetal fraction in a sample from a pregnant female is obtained by obtaining the read counts from one or more polymorphisms in a sample, and determining whether a polymorphism is informative based on these read counts and polymorphism specific attributes, whereby the read count of each informative polymorphism is related to the estimated fetal fraction.

In an embodiment of the current invention, said fetal fraction in a sample is determined as follows:

- receiving the sequences of at least a portion of the nucleic acid molecules contained in a biological sample obtained from said pregnant female;

- counting the number of sequences that align to a predefined set of polymorphisms

- comparing the obtained number of sequences with the expected number of sequences for each polymorphic site to identify the informative polymorphic site(s) for the sample;

- calculating from the obtained number of sequences for said informative polymorphic site(s) an amount, whereby said amount is an indication for the fetal fraction.

Said amount is calculated using linear scaling based on informative polymorphism-specific attributes.

In one embodiment, said sequences are obtained by next generation sequencing. In a further preferred embodiment, said sequencing method is a random low coverage sequencing method.

By preference, said polymorphisms are copy number variations with a size between 100 bp and 1 Mb, or between 1 kb and 1 Mb, or between 2 bp and 10 Mb.

Said fetal fraction may serve as an internal quality control of the sample and thus accounts for another secondary parameter obtained from said sample.

In another embodiment of the current invention, a method for determining the fetal fraction is based on cases in which a male pregnancy (i.e. the pregnant woman carries a male fetus) was identified. If a male pregnancy has been detected, the fetal fraction can be determined on the basis of reads aligned to the Y chromosome. The X and Y chromosome typically have regions that are similar between X and Y chromosome, called Pseudo-Autosomal-Regions (PAR). As the Y chromosome is small in size, the influence of these PAR regions is strong, as only a minor amount of the reads will be attributed to specific regions within the Y chromosome. The influence of PAR on the X chromosome is of lesser importance due to the size of the X chromosome.

In a first step, those regions that are unique for the X or Y chromosome (outside the PAR regions) are defined. Reads directed against these unique X and/or Y chromosome regions are counted and the fetal fraction is determined on the basis of the unique X and/or Y chromosome regions (or a derivative of these reads, such as normalised reads).

Based on the X-chromosomes, fetal fraction can be computed as twice the difference at the 50kb bin level between the median number of reads mapping to the autosomes and the median number of reads mapping to chromosome X, divided by the median number of reads mapping to the autosomes. This can be written as the following formula:

$$FF_X = 2 \times \left| 1 - \frac{\underset{\text{on } X}{\text{median}}(GC - \text{corr counts})}{\underset{\text{on autosomes}}{\text{median}}(GC - \text{corr counts})} \right|$$

Secondly, fetal fraction can also be estimated based on the Y-chromosome as all reads that map to chromosome Y should in theory originate from the fetal DNA. Chromosome Y-based fetal fraction is defined as twice the median number of GC-corrected reads mapping to Y over the median number of GC-corrected reads mapping to the autosomes, or in a formula:

$$FF_Y = 2 \times \frac{\underset{\text{over } Y}{\text{median}}(GC - \text{corr counts})}{\underset{\text{on autosomes}}{\text{median}}(GC - \text{corr counts})}$$

The current invention equally provides for a computer program product comprising a computer readable medium encoded with a plurality of instructions for controlling a computing system to perform an operation of determining or estimating the fetal fraction in a biological sample obtained from a pregnant female subject according to the current invention. Specifically, the operation comprises the steps of:

- receiving the sequences of at least a portion of the nucleic acid molecules contained in a biological sample obtained from said pregnant female;

- counting the number of sequences that align to a predefined set of polymorphisms

- comparing the obtained number of sequences with the expected number of sequences to identify the informative polymorphic site(s) for the sample; and

- calculating from the obtained number of sequences for said informative polymorphic site(s) an amount, whereby said amount is an indication for the fetal fraction.

VI Gender determination

Gender determination can occur by determining regions which are informative or indicative for male pregnancies. These regions can be defined by looking into a dataset comprising sequencing data from male datasets. Regions that are statistically indicative for male pregnancies are subsequently withhold.
The reads for one or more informative regions - which typically reside on the Y chromosome - are obtained from the analyzed sample and compared to a predefined threshold value. If the total number of reads across all selected regions in a test sample is above a first threshold value, it is most possibly a male pregnancy. On the other hand, if the total number of reads across all selected regions in a test sample is below a second threshold value, it is most possibly a female pregnancy. If the total number of reads across all selected regions in a test sample is in between the first and the second threshold value, the gender is undetermined (this could be the case for vanishing twins). By proper selection of the regions, the first and the second threshold values, the method would be less sensitive towards bias resulting from vanishing twins (especially vanishing boys).

VII Toolbox and kit

By preference, the methodologies as described above are all computer implemented. To that purpose, the current invention equally relates to a computer program product comprising a computer readable medium encoded with a plurality of instructions for controlling a computing system to perform an operation for performing prenatal diagnosis of a fetal aneuploidy and/or screening for fetal aneuploidies and/or determination of the fetal fraction in a biological sample obtained from a pregnant female subject, wherein the biological sample includes nucleic acid molecules.
With regard to the determination of the presence or absence of an aneuploidy in a sample, the operation comprises the steps of:

- receiving the sequences of at least a portion the nucleic acid molecules contained in a biological sample obtained from said pregnant female;
- aligning said obtained sequences to a reference genome;
- counting the number of reads on a set of chromosomal segments and/or chromosomes thereby obtaining read counts;
- normalizing said read counts or a derivative thereof into a normalized number of reads;
- obtaining a first score of said normalized reads and a collection of scores of said normalized reads, whereby said first score is derived from the normalized reads for a target chromosome or chromosomal segment and whereby said collection of scores is a set of scores derived from the normalized number of reads for a set of chromosomes or chromosomal segments that include said target chromosomal segment or chromosome;
- calculating a parameter p from said first score and said collection of scores.

In a preferred embodiment, said parameter whereby said parameter represents a ratio between

 * a first score, corrected by a summary statistics of said collection of scores, and
 * a summary statistics of the collection of scores.

With regard to the determination of the fetal fraction within a biological sample, said operation comprises the steps of:

- receiving the sequences of at least a portion of the nucleic acid molecules contained in a biological sample obtained from said pregnant female;
- counting the number of sequences that align to a predefined set of polymorphisms
- comparing the obtained number of sequences with the expected number of sequences to identify the informative polymorphic site(s) for the sample; and
- calculating from the obtained number of sequences for said informative polymorphic site(s) an amount, whereby said amount is an indication for the fetal fraction.

In another embodiment, said fetal fraction can be determined in case of a male pregnancy on the basis of the Y chromosome.
Said operations comprise the steps of

- receiving the sequences of at least a portion of the nucleic acid molecules contained in a biological sample obtained from said pregnant female;
- determining the gender of said fetus; whereby if said fetus is male:

 * aligning said obtained sequences to a reference database;
 * identifying and counting reads specifically located in non-PAR regions of the X and/or Y chromosome;
 * calculating from said read counts an amount, whereby said amount is an indication for the fetal fraction.

Said operations can be performed by a user or practitioner in an environment remote from the location of sample collection and/or the wet lab procedure, being the extraction of the nucleic acids from the biologic sample and the sequencing.
Said operations can be provided to the user by means of adapted software to be installed on a computer, or can be stored into the cloud.
After having performed the required or desired operation, the practitioner or user will be provided with a report or score, whereby said report or score provides information on the feature that has been analyzed. Preferably, report will comprise a link to a patient or sample ID that has been analyzed. Said report or score may provide information on the presence or absence of an aneuploidy in a sample, whereby said information is obtained on the basis of a parameter which has been calculated by the above mentioned methodology. The report may equally provide information on the nature of the aneuploidy (if detected, e.g. large or small chromosomal aberrations) and/or on the quality of the sample that has been analyzed.
Alternatively, said practitioner or user may be provided with information on the fetal fraction, whereby said fetal fraction has been determined by one of methodologies of the current invention.
In another embodiment, said practitioner or user may, by virtue of the report, be provided with gender information of the fetus.
It shall be understood by a person skilled in the art that above-mentioned information may be presented to a practitioner in one report.
By preference, above mentioned operations are part of a digital platform which enables molecular analyzing of a sample by means of various computer implemented operations.

In particular, the current invention also comprises a visualization tool, which enables the user or practitioner to visualize the obtained results as well as the raw data that has been imputed in the system. In an embodiment, said visualizations comprises a window per chromosome, depicting the chromosome that has been analyzed, showing the reads per region or a score derived thereof and the scores and/or parameter that has been calculated. By showing to the practitioner or user the calculated scores and parameter together with the visual depiction of the read counts, a user may perform an additional control or assessment of the obtained results. By allowing the user to look at the data, users will be able to define improved decision rules and thresholds.

Moreover, an additional control is added, as the visual data per chromosome enables the user to evaluate for every chromosome if the automated classification is correct. This adds an additional safety parameter.

In a preferred embodiment, said platform and visualization tool is provided with algorithms which take into account the fact that certain regions give more reads (due to a recurrent technical bias that makes some regions of the genome always over- or under-represented). Correction measures may be provided for this overrepresentation by making a comparison with a reference set (that is ideally processed using the same or similar protocol) and plotting e.g. Z scores or alternative scores that represent the unlikeliness of certain observations under the assumption of euploidy. Standard visualization tools only display read count, and do not allow to correct for the recurrent technical bias.

Finally, based on the link between the obtained scores and/or parameter and the visual data per chromosome a user or practitioner may decide to alter the threshold/cutoff value that is used to define the presence of an aneuploidy. As such, the user may decide to aim for a higher sensitivity (e.g. being less stringent on the decrease/increase of the parameter or scores) or higher specificity (e.g. by being more stringent on the increase/decrease of parameter or scores).

The platform may be provided with other features, which provide for an accurate analysis of the molecular data obtained from the biological sample.

As previously mentioned, the methodology and platform allows a certain degree of liberty to the user. Apart from defining own cutoff values and thresholds, the user may also define own reference sets of genomes, to be used to calculate the scores and/or other information such as fetal fraction, gender determination etc. By using their own reference set, a user may be allowed to better capture the recurrent technical variation of the lab (different wet lab reagents and protocol, different NGS instrument and platform, different operator, different ...) and hence have a more suitable reference data set to that particular lab. In order to ensure the robustness of a new reference set, methodologies are provided to remove outliers from the reference set. E.g. if 100 reference samples are used in a reference set, there will be 100 reference results for each 50 kb bin. If a certain predefined percentage of outliers is removed (e.g. 5% of the results), the reference set can be made more robust to variation in the reference set.

In an embodiment, a method for performing CNV calling is provided. With the term 'CNV calling' it is meant a methodology that determines the boundaries of a CNV of a CNV or a segmental aneuploidy. Said boundaries are to be understood as the approximate chromosomal coordinates.

Subsequently, these boundaries are used to cross-reference with CNV reference genome databases which contain previously observed CNVs (optionally annotated, e.g. benign or pathogenic). CNV calling can be performed by various methodologies. Some of them were developed in the array-CGH field (where one or adjacent set of aCGH probe(s) can be considered as the equivalent of a bin), others are more specific for NGS data.

In another embodiment, said platform allows CNV quantification. With the term CNV quantification is understood the determination of the absolute number of copies (or the expected range) of the observed CNV. The latter will allow determining if a CNV is rather maternal (very high value) or rather fetal (very low value). By preference, said CNV quantification is done after CNV calling. In another, more preferred embodiment, said CNV calling takes into account knowledge on the cell-free fraction.

In an embodiment, said platform allows CNV signature recognition. With the term 'CNV signature recognition' a methodology for determining whether a specific combination of CNVs (and their quantity) is present in a sample is understood. By preference, CNV signature recognition is performed after CNV calling and CNV quantification.

All methodologies mentioned above employ use of one or more CNV reference genome databases which comprise known CNVs. The methodologies mentioned above can be based on the aligning of sequences obtained from a biological sample against said one or more CNV reference databases, or alternatively aling the sequences obtained from a biological sample against a reference genome and subsequently identify the reads that were aligned to specific regions of interest (i.e. the regions identified as CNVs in the CNV reference databases). Coupling to such reference databases allow the identification of CNVs that are (likely) pathogenic and thus the (clinical) accuracy is increased: if a CNV is observed, and it contains a (likely) pathogenic region, it could be very relevant and requiring follow up of the patient.

The platform according to the current invention will allow a large degree of liberty to the user or practitioner. By preference, said platform will be compatible with various data formats such as fastq, fastq.gz, bcl and bam files. In a further embodiment, said platform is able to receive data formats which are directly streamed from the sequencing platform that is used (e.g. NGS instrument). The latter strongly reduces the waiting time for data upload as the upload happens simultaneously with the sequencing reaction. As such, the total time-to-result will be optimized, which is beneficial for the user.

In an embodiment, said platform is compatible with sequencing data from various sources, including SMRT (single molecule real time) sequencing data. Algorithms can be present that enable the processing of SMRT data (as e.g. PacBio) and deduce epigenetic information (e.g. methylation or other modifications) from said SMRT data. The latter again allows identification of parameters that indicate aneuploidy detection, or could aid in the determination of the fetal fraction, or determine quality metrics.

The platform according to the current invention is inherently compatible with many different types of NGS library preparation kits and protocols and NGS sequencing platform. This is an advantage as a user will not have to invest in dedicated NGS sequencing platform or NGS library preparation kits that are specific for a specific application, but instead a user can use its preferred platform and kit. Moreover, this allows a user a certain degree of flexibility in material to be used. If newer or cheaper instruments or kits become available, a user will be allowed an easy change.

As mentioned before, the current methodology is compatible with cell-free DNA extracted from various sorts of biological samples, including blood, saliva, blastocoel fluid and urine. Using urine or saliva instead of blood would represent a truly non-invasive sample type and allows for e.g. home-testing and shipment of the sample to the test lab. This is obviously an additional advantage compared to other sample obtaining methods such as drawing blood.

**Examples**

Preparation and sequencing of the sample

1. Blood collection, plasma separation and cell-free DNA extraction

One tube (10 ml) of maternal blood is collected in Streck tubes and stored at 4°C. The blood is collected via a standard phlebotomy procedure.

The plasma (+/- 5 ml) is separated maximum 72 hours after blood sampling by the standard dual centrifugation method:

- The blood sample is centrifuged at 2000xg for 20 minutes (this may be done at room temperature), without using the brake.
- The plasma is then transferred to either three 1.5ml low binding tubes, or to a single 5 ml low binding tube. A second centrifugation at 13,000xg is done for 2 minutes (this may be done at room temperature).
- The plasma is transferred to sterile 1.5 ml or 5 ml low binding tubes for storage at -20°C prior to cell-free DNA (cfDNA) extraction.

Optionally, the buffy coat layer can be stored for future testing. Maternal genomic DNA from the buffy coat layer can be investigated to confirm or exclude maternal abnormalities.

The cell-free DNA is extracted from the plasma using the QIAamp Circulating Nucleic Acid Kit (Qiagen) according to the manufacturer's recommendations, with a final elution volume of 60 $\mu$l. The DNA samples are stored at -20°C when not used immediately for library preparation.

2. cfDNA quantification

The extracted cfDNA is quantified using a Qubit fluorometer. The cell-free DNA concentration is usually around 0.1-1 ng/$\mu$l.

3. Library preparation

25 $\mu$l of the extracted cfDNA is used as starting material for library preparation. During library preparation, the DNA samples are adapted for next-generation sequencing. Adaptors are added to the ends of the DNA fragments. The sequencing libraries are prepared using the TruSeq ChIP library preparation kit (Illumina) with some adaptation of the manufacturer's protocol by reducing the reagent volumes to allow the generation of sequencing libraries using low starting amounts of DNA.

The library preparation protocol may be summarized as follows:

(Note: The bead-based size selection for removal of large DNA fragments and removal of small DNA fragments described in the protocol is NOT used.)

<u>End repair of the DNA fragments:</u>

1. Add 5 µl Resuspension Buffer and 20 µl End Repair Mix to the 25 µl starting material (total = 50 µl)
The bead-based size selection for removal of large DNA fragments and removal of small DNA fragments described in the protocol is NOT used.
2. Incubate 30 minutes at 30°C
3. Add 80 µl undiluted AMPure beads to the 50 µl sample mixture after end repair.
4. Wash the beads twice with 190 µl 80% EtOH
5. Resuspend the dried pellet with 10 µl Resuspension Buffer. Transfer 9 µl of the supernatant to a new tube.

<u>Adenylation of the the 3' ends</u>

1. Add 6.25 µl A-tailing Mix
2. Heat 30 minutes at 37°C + 5 minutes at 70°C

<u>Ligation of the indexed paired-end adaptors to the DNA</u>

1. Adaptors are diluted 1/2 with Resuspension Buffer => add 2,5 µl to sample
2. Add 1.25µl Ligation Mix (no Resuspension Buffer). Incubate 30 minutes at 30°C
3. Add 2.5 µl Stop ligation buffer
4. Add 21 µl AMPure beads for clean-up
5. Wash the beads twice with 190 µl 80% EtOH
6. Resuspend the dried pellet in 27 µl Resuspension Buffer. Transfer 25 µl of the supernatant to a new tube.
7. Add 25 µl AMPure beads for clean-up
8. Wash the beads twice with 190 µl 80% EtOH
9. Resuspend the dried pellet in 12.5 µl Resuspension Buffer. Transfer 10 µl of the supernatant to a new tube.

<u>Enrich DNA fragments</u>

1. Prepare the PCR mix by mixing 2,5 µl PCR Primer Cocktail and 12,5 µl PCR Master Mix for each sample.
2. PCR conditions:

98°C for 30 seconds
15 cycles of:

98°C for 10 seconds
60°C for 30 seconds
72°C for 30 seconds
72°C for 5 minutes
hold at 4°C

3. Add 25 µl of AMPure beads for clean up
4. Wash the beads twice with 190 µl 80% EtOH
5. Resuspend the dried pellet in 32.5 µl Resuspension Buffer. Transfer 30 µl of the supernatant to a new tube.
6. Use 2 µl of the sample for Qubit quantification and 2µl for fragment analysis (see next section)

<u>4. Library quality check</u>

Proper cell-free DNA isolation and NGS library preparation are tested by analyzing every library on the Fragment Analyzer (Advanced Analytical Technologies Inc., Germany) prior to sequencing, to assess:

- the size distribution (confirm suitable size profile using concentration, peak ratio, peak height, ...),
- the quality of the library. Samples containing high molecular weight fragments will be classified as not eligible for sequencing (indicates contamination with maternal genomic DNA).

Typical libraries demonstrate a narrow size distribution with a peak at about 300-350 bp.
Additionally a Qubit quantification step is performed so that the enrichment reaction is done with the appropriate amount of input DNA material. DNA concentration is usually around 15-30 ng/μl.

5. Normalize and pool libraries

Samples are indexed during library preparation and up to 24 samples are normalized and pooled in equal volumes for multiplex sequencing across both lanes of an Illumina HiSeq2500 flow cell.

6. NGS run

Sequencing is performed on the HiSeq 2500 (Illumina) in Rapid Run mode producing 50bp single end reads.

Detection of an aneuploidy in a biological sample: validation of the methodology

* Mapping and filtering of the mapped reads

The 50bp single end sequence reads of a test sample are mapped to the reference genome GRCh37.75 with BWA-backtrack. With Picard tools duplicated reads are removed and based on the mapping quality reads mapping to multiple locations are discarded. Also reads mapping sub-optimally to multiple locations are removed. To reduce sample variability, we retain only those reads that match perfectly with the reference genome (i.e., no mismatches and no gaps are allowed). Finally, also reads that fall in an in-house curated list of blacklisted regions are removed. These blacklisted regions comprise common polymorphic CNVs, collapsed repeats, DAC blacklisted regions generated for the ENCODE project and the undefined portion of the reference genome (i.e., the Ns).

* Computing genomic representation

The reference genome is divided in bins of 50kb and the number of reads of the test sample is counted per bin. These read counts are corrected according to the GC contents of the bins with locally weighted scatterplot smoothing (Loess regression). These GC-corrected read counts are then divided by the total sum of all autosomal GC-corrected read counts and multiplied by $10^7$. This is defined as the genomic representations (GR) per bin. On these per-bin GR values a sliding window is applied and the sum of these GR values is computed for all consecutive 100 bins. The windows are each time shifted with 1 bin (i.e., 50 kb). In this way a GR value is obtained per 5Mb window. Similarly, for each autosome also the sum of the per-bin GR values is calculated, to obtain a GR value for each autosome in the test sample.

* Comparison with a reference set

In a reference set of 100 *normal* samples (50 male and 50 female pregnancies) the GR values are computed for all autosomes and for all 5Mb windows as described above. For each autosome and 5Mb window the mean $\mu$ and the standard deviation $\sigma$ of the GR scores are computed over all 100 reference samples. This allows computing a *Z-score* for each window and each autosome *i* in a test sample, defined as

$$Z_i = \frac{GR_i - \mu_i}{\sigma_i}$$

where $GR_i$ is the GR value in the test sample for window or autosome *i* and $\mu_i$, $\sigma_i$ the average and standard deviation, respectively, of the GR scores measured in the 100 reference samples for window or autosome *i*.
Based on the 22 Z-scores of the autosomes in a test sample, a *ZZ2 score* is computed for each autosome as

$$ZZ2 = \frac{Z_i - \underset{j=1,\dots,22}{\mathrm{median}}(Z_j)}{\underset{j=1,\dots,22}{\mathrm{sd}}(Z_j)}$$

where the Z-score $Z_i$ of autosome *i* in the test sample is compared with the median and the standard deviation (sd) of the 22 Z-scores obtained for all 22 autosomes in the test sample.

Alternatively, a *ZofZ-score* is computed as

$$Z \text{of } Z_i = \frac{Z_i - \underset{j=1,\dots,22}{\text{median}}(Z_j)}{\underset{j=1,\dots,22}{\text{mad}}(Z_j)}$$

where the Z-score $Z_i$ of chromosome *i* in the test sample is compared with the median and the median absolute deviation (mad) of the 22 Z-scores obtained for all 22 autosomes in the test sample. The ZZ2 and ZofZ-scores quantify the deviation of the Z-score of the target autosome from all Z-scores observed in the test sample. This robust version of the Z-of-Z scores does not make any assumptions on the aneuploidy state of the autosome under consideration and the other autosomes.

Based on the Z-scores computed for all 5Mb windows in the test sample, the *BM score* of each autosome i is computed as the median of the Z-scores over all windows of the target autosome:

$$\text{BM}_i = \underset{j=\text{window on i}}{\text{median}}(Z_j)$$

where the median of the Z-scores is computed over all windows j on autosome *i*.

This BM-score reflects the size of the aberration: aneuploidies will result in large BM values, while smaller, segmental CNVs will have less influence on the median of the Z-scores and result in smaller BM-scores.

To distinguish high, aberration-related BM scores from augmented BM values due to noise in the data set, the OM score for an autosome i is computed as the median of the Z-scores of all windows of the other autosomes:

$$\text{OM}_i = \underset{j=\text{window not on i}}{\text{median}}|Z_j|$$

where the median is computed over all, absolute Z-scores obtained for 5Mb windows j not located on autosome i.

Finally, for each test sample a *quality score (QS)* is calculated as

$$QS = \underset{j}{\text{sd}}(Z_j)$$

with j over all autosomes expect for the 2 autosomes with the highest and the lowest Z-score. This score will identify test samples of poor quality that will result in unreliable aneuploidy calling. A highly elevated QS-score can also hint at DNA samples containing at least a fraction of DNA originating from a tumor.

For each of the above calculated parameters, a threshold value can be defined. Based on standard statistical considerations, one could choose a threshold value of 2, 2.5 or 3. In the context of the Z-score, this means that the chance that the test result is normal (i.e. the obtained GR score is similar as the GR scores for the same region in the reference set) is very unlikely. In order to make a test more specific, one could increase the threshold value. In order to make a test more sensitive, one could decrease the threshold value. These threshold values can be defined for each of the parameters, and can differ for each of the parameters. It is for instance conceivable that threshold values for BM and OM are set to 1, while for Z score and ZZ-score they are set to 3. Also negative threshold values can be used.

**Example** 1

1.1 ZZ2 computation for chromosome 21 for sample A

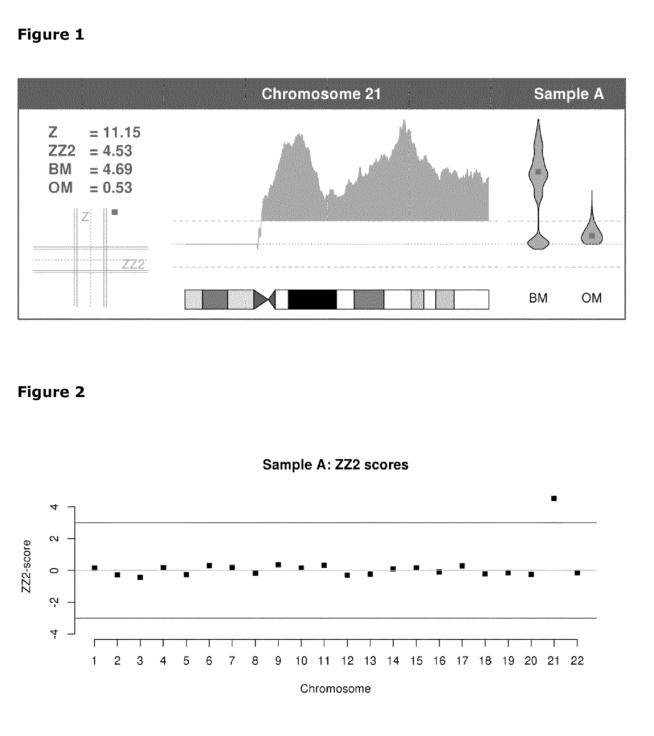

For sample A, the median of the Z-scores for all chromosomes equals -0.1641 and the standard deviation equals 2.494. For chromosome 21, we have a Z-score of 11.147 and this results in a ZZ2 score of 4.5347, above the threshold of 3. (Figure 1)

This automated classification as an abnormal chromosome based on ZZ2>3, is also confirmed by visual inspection of the plot. This sample was tested with an invasive test and the trisomy was confirmed.

None of the other chromosomes in sample A have elevated ZZ2 scores (figure 2).

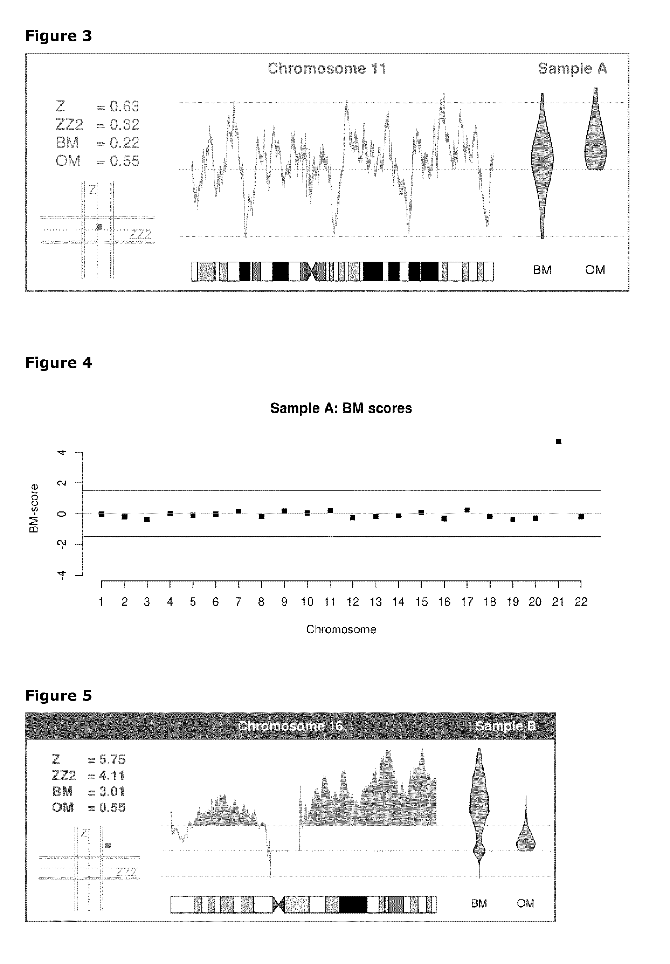

The plots of the Z-scores of these other chromosomes are also not indicative for aneuploidy. For example, on chromosome 11 we have a ZZ2 score 0.319 and this results in the plot as shown in figure 3.

The plot in figure 4 shows the BM scores of all autosomes. The latter confirms that the chromosome 21 is strongly aberrant, while other potential autosomal aberrations will span less than half of the chromosome.

1.2 ZofZ computation for chromosome 21 for sample A

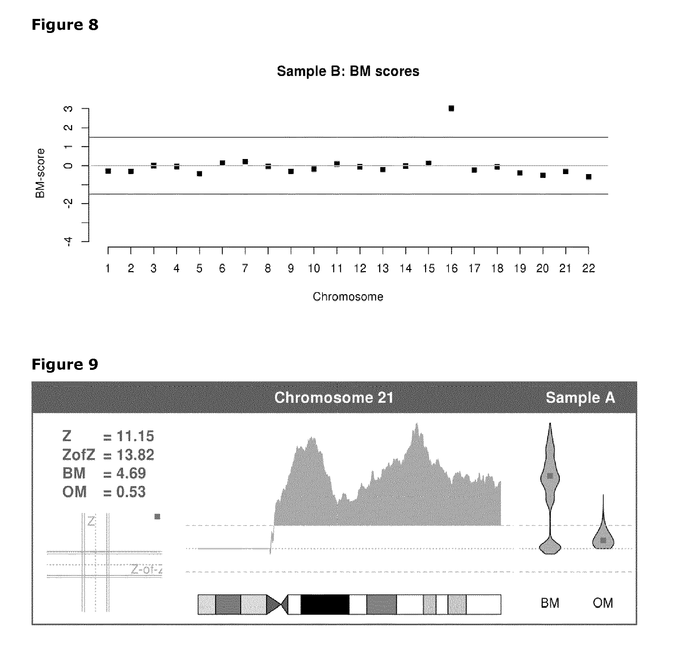

For sample A, the median of the Z-scores for all chromosomes equals -0.164 and the median absolute deviation equals 0.819. For chromosome 21, we have a Z-score of 11.147 and this results in a ZofZ-score of 13.817, far above the threshold of 3. If we plot the Z-scores, this clearly hints at a trisomy 21 (figure 9). This sample was tested with an invasive test and the trisomy was confirmed.

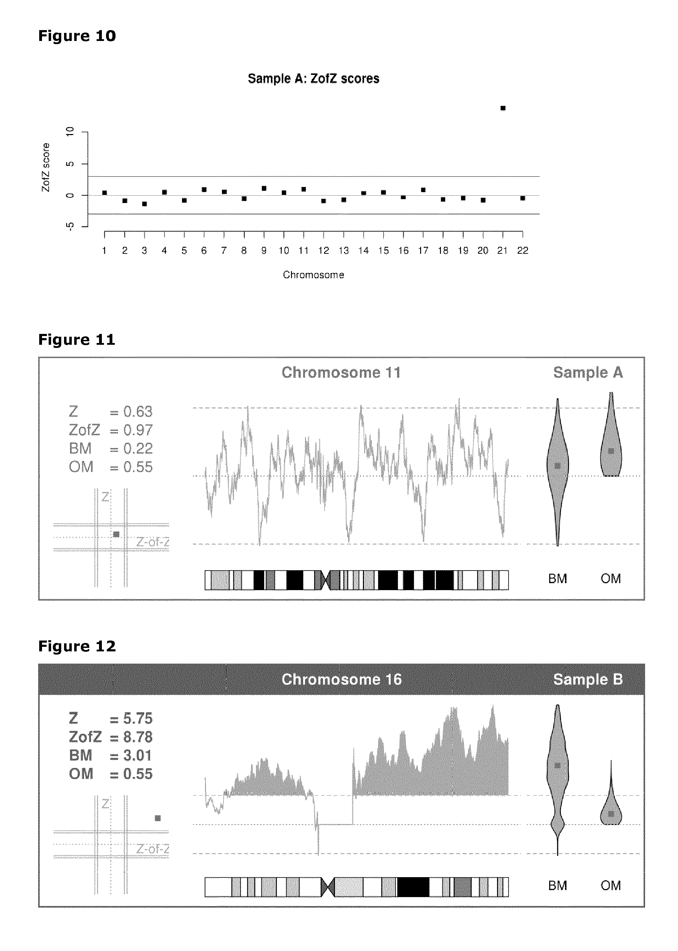

Based on these ZofZ scores and a threshold of 3, none of the other chromosomes would be called aneuploidy (figure 10).

The plots of the Z-scores of these other chromosomes are also not indicative for aneuploidy. For example, on chromosome 11 we have a ZofZ-score 0.973 and this results in the plot as shown in figure 11.

1.3 ZZ2 computation for chromosome 16 for sample B

In sample B the median of the Z-scores for all chromosomes equals -0.2651 and the standard deviation equals 1.464. For chromosome 16, we have a Z-score of 5.754 which results in a ZZ2 score of 4.111, which is above the threshold of 3.

Indeed, if the Z-scores are plotted (figure 5), this clearly hints at a trisomy 16. This sample was tested with an invasive test and the trisomy was confirmed.

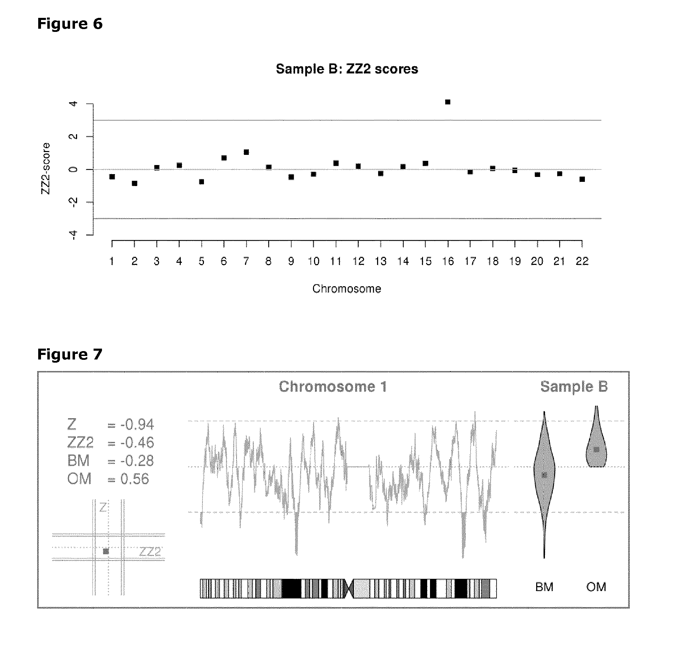

None of the other chromosomes in sample B have elevated ZZ2-scores (figure 6).

The plots of the Z-scores of these other chromosomes are also not indicative for aneuploidy. For example, on chromosome 1 we have a ZZ2 score -0.459 which results in the plot shown in figure 7.

The plot of the BM scores of all autosomes as shown in figure 8 confirms that chromosome 16 is strongly aberrant, while other, potential autosomal aberrations will span less than half of the chromosome.

1.4 ZofZ computation for chromosome 16 for sample B

For sample B, the median of the Z-scores for all chromosomes equals -0.265 and the median absolute deviation equals 0.685. For chromosome 16, we have a Z-score of 5.754 which results in a ZofZ-score of 8.782, above the threshold of 3. Indeed, if we plot the Z-scores, this clearly hints at a trisomy 16 (figure 12). This sample was tested with an invasive test and the trisomy was confirmed.

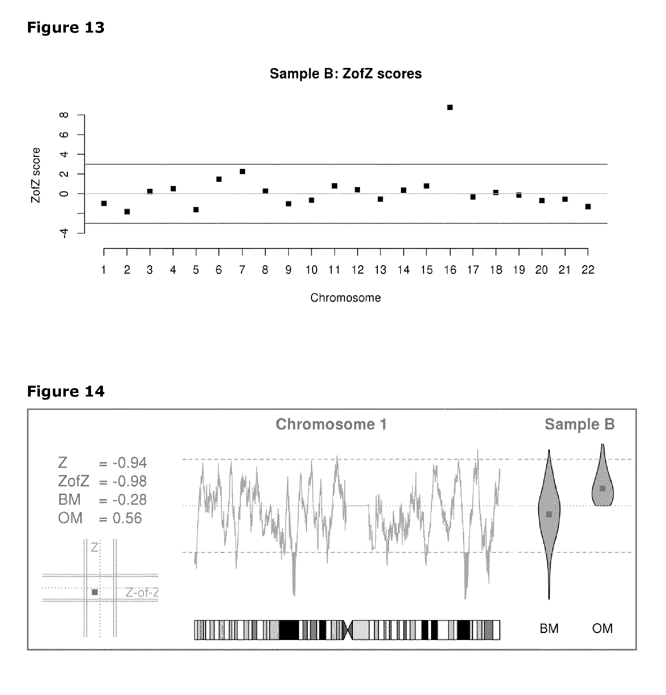

Based on these ZofZ-scores and a threshold of 3, none of the other chromosomes would be called aneuploidy (figure 13).

The plots of these other chromosomes are also not indicative for aneuploidy. For example, on chromosome 1 we have a ZofZ-score -0.980 (figure 14).

1.5 The sample quality assessment via OM and QS

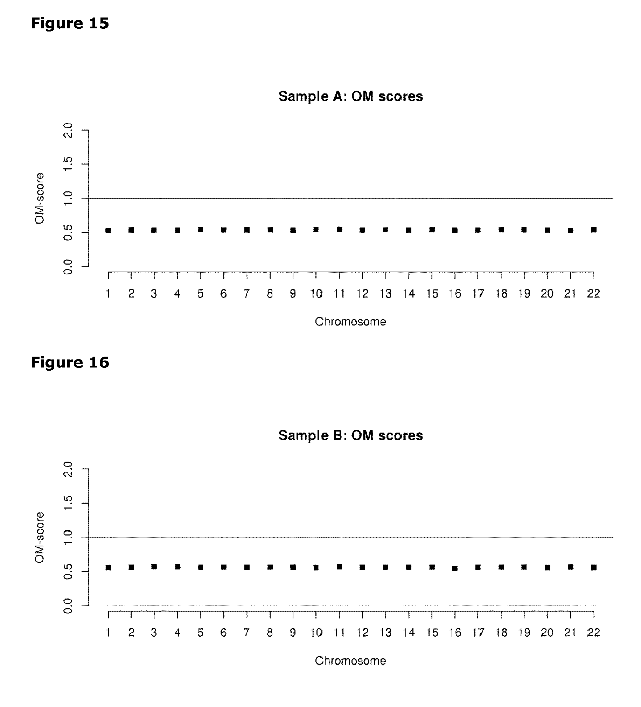

Samples A and B were two clear cases in which one chromosome is trisomic, while all other autosomes behave diploid. The following plots show the OM values of the autosomes in samples A and B and all OM values confirm that this was a successful experiment. This is also confirmed by low QS scores (i.e., 0.576 for sample A and 0.652 for sample B, see figure 15 and 16)).

**Example** 2

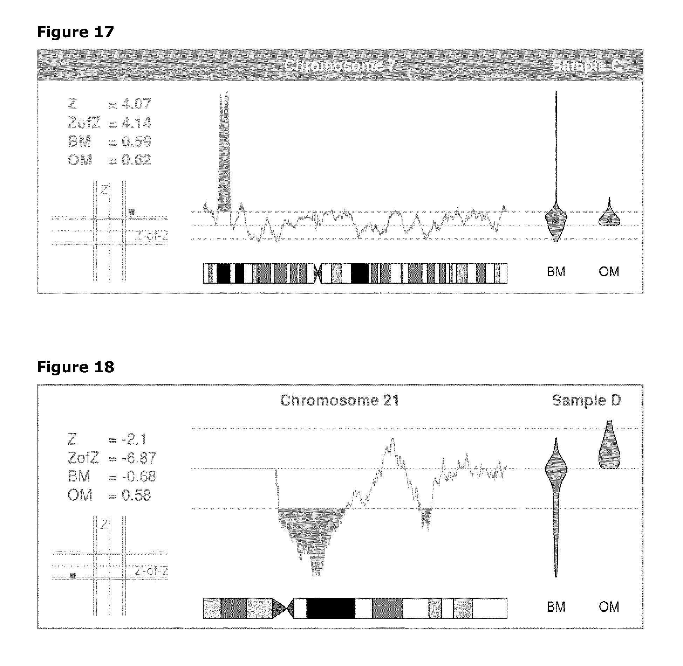

In sample C we observe a ZofZ-score of 4.141 for chromosome 7, while the BM score remains low. Figure 17 shows a plot of chromosome 7, where a part of the chromosome that is likely to have higher copy number is observed. This can indicate a maternal CNV.

Hence, ZofZ scores can also be indicative for segmental CNVs

Automated classification of the chromosome using ZofZ>3 is very sensitive for picking up CNVs (see Sample C) or larger aneuploidies (see Sample A and B). Hence ZofZ can be used to identify abnormal chromosomes, and visual inspection of the plot can confirm the presence of such abnormalities.

Automated classification of the chromosome using a combination of ZofZ>3 with another parameter, can further improve the specificity of the automated classification, and add more granularity to the results. If e.g. ZofZ>3 and BM<1, this can indicate the presence of a CNV (see Sample C), while if e.g. ZofZ>3 and BM>1, this can indicate the presence of larger aneuploidy (see Samples A and B).

## Example 3: ZofZ more sensitive than ZZ

The ZofZ-score can be more sensitive as compared to ZZ for the identification of CNVs. ZofZ-score can also be more sensitive as compared to ZZ for the identification of CNVs or larger aneuploidies in noisy samples.

3.1 Segmental aberration on chromosome 21

In sample D a ZofZ-score of -6.873 for chromosome 21 was observed, while the ZZ score as described in Bayindir et al. 2015 (which was used as reference method) resulted in a ZZ-score of -2.341 (i.e., not significant). In the plot of that chromosome as shown in figure 18, we observe a part of the chromosome that seems to be underrepresented. This can indicate a maternal or a fetal CNV.

3.2 Segmental aberration on chromosome 15

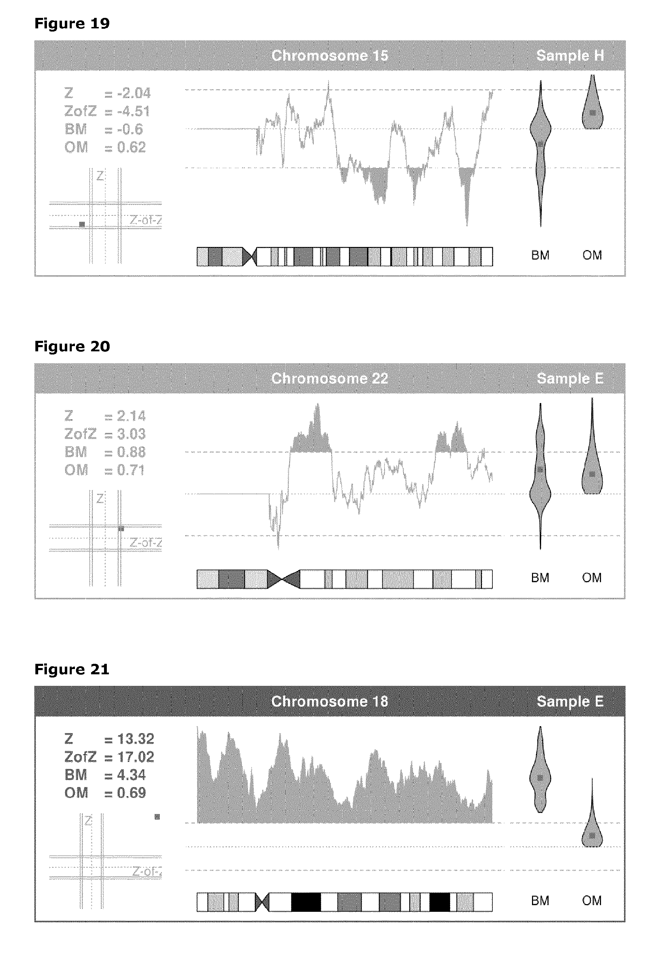

Based on the ZZ-score and the decision tree described in Bayindir et al. 2015, this chromosome 15 would be classified as a normal profile (ZZ =-2.730). The ZofZ score of -4.51 however draws attention to this chromosome (see figure 19). The BM score equals -0.6, which indicates that the aberration is partial, as could also be deduced from visual inspection of the plot.

3.3 Segmental aberration on chromosome 22

In sample E a ZofZ-score of 3.029 was observed for chromosome 22 (see figure 20), while the ZZ score as described in Bayindir et al. 2015 resulted in a ZZ-score of -0.629 (i.e., not significant).
Based on the ZZ-score and the decision tree described in Bayindir et al. 2015, this chromosome would not be flagged and classified as normal. This could be due to the fact that this sample also has a trisomy 18 (see figure 21).

3.4 Segmental aberration on chromosome 20

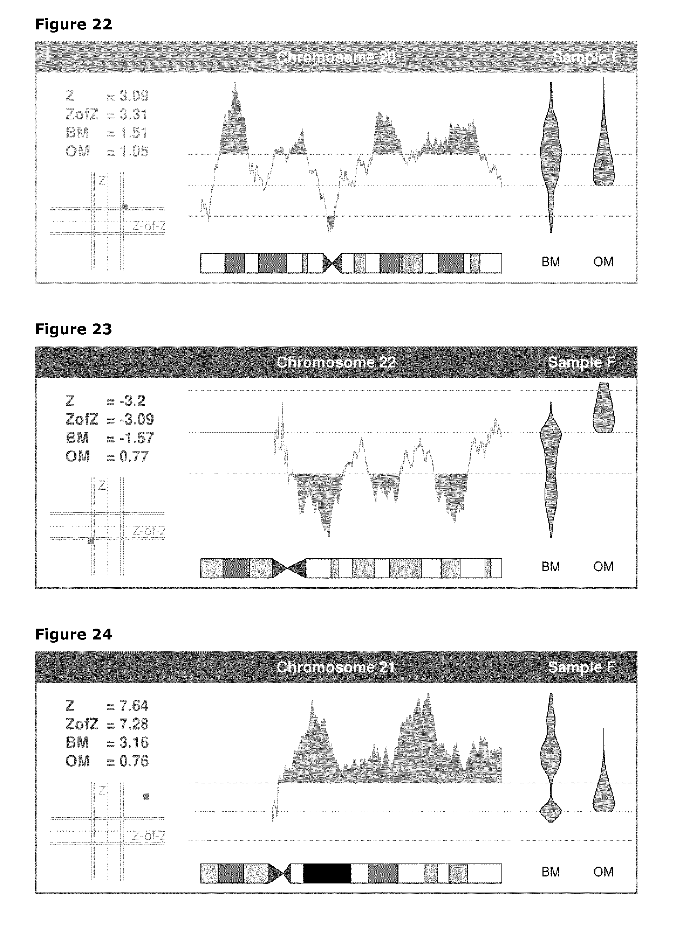

In sample I, the ZZ-score as described in Bayindir et al. 2015 would be less than 3 (i.e., ZZ=2.195), while the ZofZ-score equals 3.31. Also the BM score equals 1.51, indicating that more than half of the Z-scores are increased. The OM score of 1.05 shows that this dataset was rather noisy (see figure 22).

3.5 Indication for monosomy of chromosome 22

In sample F we observe a ZofZ-score of -3.094 for chromosome 22, while the ZZ score calculated as described in Bayindir et al. 2015 equals -1.771 (i.e., not significant) (figure 23). The ZZ score would result in a normal value. Sample F has a trisomy 21 that perhaps masks this monosomic behavior of chromosome 22. Note that the trisomy 21 was confirmed via invasive follow-up. There were no follow-up data for chromosome 22.
The latter results all show that the method according to the current invention is more sensitive than the methodologies known to date. The visual representation of the data as shown in the figures allows automated classification and interpretation. Moreover, the visualization according to the current invention allows distinguishing between technical noise and noise which is due to aneuploidies.
If the data are noisy, and vary along the Z=0 axis (i.e. both higher and lower than 0), then this is more likely to be technical noise. While if the data do not vary along the Z = 0 axis for a large chromosomal segment, this is more likely to be a real aneuploidy. From the plot in figure 24, it is obvious that the view is not due to technical noise but due to an aberrant situation.

## Example 4: Gender determination

The gender of the samples can be determined by assessing the number of reads mapping to twenty 50kb bins on chromosome Y that were empirically selected to be specific for male pregnancies (see Bayindir 2015 for further details). In case at least 3 or more Y-specific bins contain more than 1 read, the gender was determined to be male. In case at most 1 bin contained 1 read or none of the 20 bins contained a read, the gender was said to be female. In all other cases, no gender specification was done and the gender was said to be undetermined. This could be due to e.g. a vanishing twin; where the blood sample contains fetal DNA of two fetuses instead of one.

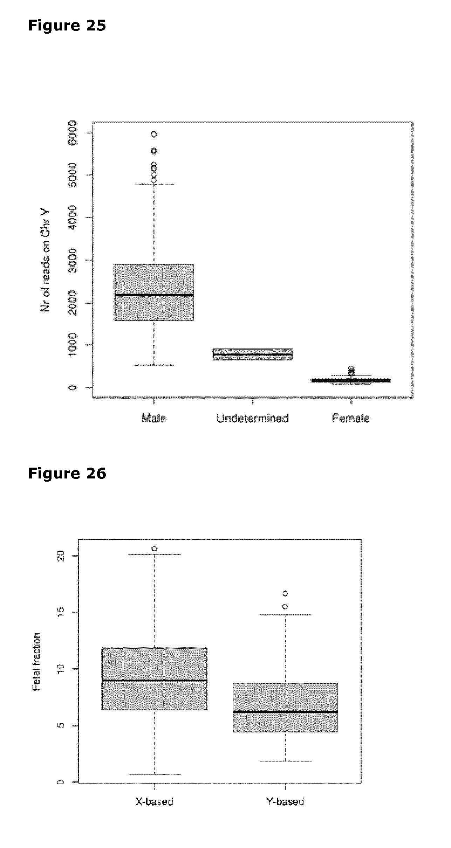

In a set of 249 succeeded experiments (i.e., QS-score was lower than 1.5 and the number of reads remaining after all filtering steps was higher than 7,000,000), the gender was determined and this resulted in a set of 116 (46.59%) female pregnancies, 131 (52.61%) male pregnancies and 2 (0.80%) undetermined pregnancies. The plots as shown in figure 24 and 25 for these 249 samples show the number of reads that mapped to the Y-chromosome (after filtering the BAM file).

4.1 Fetal fraction determination for male pregnancies

Once the gender is determined, fetal fraction can be determined for the male pregnancies. For male pregnancies, one can benefit from the fact that the fetal DNA will only have 1 copy of X and a copy of Y, instead of the 2 copies of X that are present in the maternal DNA. This allows estimating the fetal fraction in two ways. Based on the X-chromosomes, fetal fraction can be computed as twice the difference at the 50kb bin level between the median number of reads mapping to the autosomes and the median number of reads mapping to chromosome X, divided by the median number of reads mapping to the autosomes. This can be written as the following formula:

$$\mathrm{FF_X} = 2 \times \left| 1 - \frac{\underset{\mathrm{on}\ X}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})} \right|$$

Secondly, fetal fraction can also be estimated based on the Y-chromosome as all reads that map to chromosome Y should in theory originate from the fetal DNA. Chromosome Y-based fetal fraction is defined as twice the median number of GC-corrected reads mapping to Y over the median number of GC-corrected reads mapping to the autosomes, or in a formula:

$$\mathrm{FF_Y} = 2 \times \frac{\underset{\mathrm{over}\ Y}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})}$$

Disadvantage of this approach is that fetal fraction can only be determined for about half of the samples (i.e. only for the male pregnancies). In our previous examples fetal fractions were computed. Results are shown in the Table I.

| Sample | Sample description | Gender | FF$_X$ | FF$_Y$ |
|---|---|---|---|---|
| **A** | Trisomy 21 sample | Male | 8.6135 | 7.4662 |
| **B** | Trisomy 16 sample | Female | | |
| **C** | Maternal CNV on chr 7 | Female | | |
| **E** | Trisomy 18 and segmental aberration chr 22 | Female | | |
| **D** | Segmental aberration chr 21 | Female | | |
| **F** | Trisomy 21 and monosomic behavior for chr 22 | Male | 7.5631 | 6.3376 |
| **G** | Failed sample (QS = 27.526) | Male | 88.1395 | 48.7756 |
| **H** | Segmental aberration chr 15 | Male | 17.8092 | 15.9849 |
| **I** | Segmental aberration chr 20 | Female | | |

Figure 25 shows for the 131 male pregnancies, identified in example 4.1, the X and Y-based fetal fractions.

**Example 6: CNV based approach to determine a minority fraction (e.g Fetal fraction)**

The methodology of the current invention can be exemplified if one assumes a sample with a fetal fraction of 10%, that was sequenced at a coverage of 0.1X to yield 50 bp reads. Further one should assume a CNV of 10 Mb.

In case of a normal sample (i.e., 2 copies of the CNV) consisting out of 100% DNA of the patient, 20,000 reads mapping to that CNV region is expected. In case the patient has only 1 copy of the particular CNV, 10,000 reads are expected in that region; in case of 0 copies, no reads are expected.

In case of a minority fraction of 10%, the following cases can be expected (Table II):

**Table II**

| # copies woman | # copies fetus | Expected # reads |
|---|---|---|
| 2 | 2 | ~20,000 |
| 2 | 1 | ~19,000 |
| 2 | 0 | ~18,000 |
| 1 | 2 | ~11,000 |
| 1 | 1 | ~10,000 |
| 1 | 0 | ~9,000 |
| 0 | 2 | ~2,000 |
| 0 | 1 | ~1,000 |
| 0 | 0 | ~0 |

In case the CNV is found with far too low, non-zero coverage, it can be concluded that the mother does not have this region (0 copies) and that the reads which are observed come from the minority DNA. Hence the CNV can be considered to be informative for the determination of the fetal fraction.

Hence the minority fraction can be determined as:

$$2*\text{observed reads/expected reads} = 2*1,000/20,000 = 10\%$$

As an estimate for the expected number of fragments, the global coverage of the sample can be used as well as the length of the CNV. A correction for overall read depth can occur and the expected coverage based on all samples can be computed, by e.g. taking the mean after excluding the top and bottom 10% of the samples or by using the median coverage.

In order to correct for recurrent technical noise, a CNV specific attribute can be calculated using samples with known fetal fraction, in which the attribute can correct the obtained read counts for recurrent technical noise on that particular CNV.

The sequences can be obtained as described above, except that filtering of reads that lie in blacklisted regions was not applied.

Computing coverage per CNV

A CNV reference genome database was used, containing 581774 CNVs. Note that the CNVs in this set were not all unique CNVs as the database contains overlaps.

The reads were aligned against the reference genome. For each CNV, the number of reads was counted that showed overlap of at least X bases with the CNV regions, whereby X can be any value between 1 and 50. In the current example, X was set to 1. This results in a matrix with the raw counts. Raw counts equal or less than X are defined as equal to 0, as they display minimal overlap with the CNV.

Because the raw counts were not corrected for the total number of reads, the total number of filtered reads was extracted for each sample and was corrected by the following equation:

$$\text{normalized count} = 10,000,000 * \text{raw count/total number of reads;}$$

whereby 10,000,000 was arbitrary chosen and could be replaced by any other value.

Previously determined fetal fractions obtained for a set of male pregnancies were imported. These fetal fractions are based on the counts on the X and Y chromosome and use the fact that in male pregnancies the fetal DNA only has 1 copy of chromosome X and one copy of chromosome Y; while the maternal DNA has two copies of chromosome X. Hence the X and Y-based fetal fraction method can only give an estimate on the fetal fraction for the male pregnancies (as opposed to the CNV based fetal fraction method presented here).

The previously obtained fetal fractions were filtered to remove samples with a fetal fraction smaller than Y. Y can be any value between 0.05 and 1. In the current example said Y was set to 0.3, as this is considered to be the maximal fetal fraction that one would routinely encounter

Determining of informative CNVs in a set of CNVs

In a subsequent step, informative CNVs were determined. For each CNV, samples were identified for which only the fetus had 1 or 2 copies and whereby the mother had no copies. All counts are thus derived from the fetus. Next it was checked whether for the male pregnancies these fetal counts correlate to the X/Y based fetal factions, thereby resulting in a X-based and Y-based correlation. This gave insight in which CNVs were informative for fetal fractions.

As an example, the following 3 random CNVs were considered, covering different lengths:

1. Chr 1: 72,773,259-72,798,581; this is a region of 25kb (25,323bp)
2. Chr 1: 148,539,255-149,765,886; this is a region of 1Mb (1,226,632bp)
3. Chr 9: 38,725,590-71,025,693; this is a region of 32Mb (32,300,104bp)

In a histogram (not shown) of the normalized counts (normalized with regard to the total number of reads) for CNV 1, 3 peaks are observed (likely to be corresponding to 0, 1 and 2 maternal copies). For CNV 2, 4 large peaks were observed (likely to be corresponding to 0, 1, 2 and 3 maternal copies). In the third and largest CNV no peaks were observable. Therefore, analysis is continued with CNV 1 and 2.

To find the local minima, a density function was fitted and the signs of the derivative of the density functions were checked.

The two local minima were at:

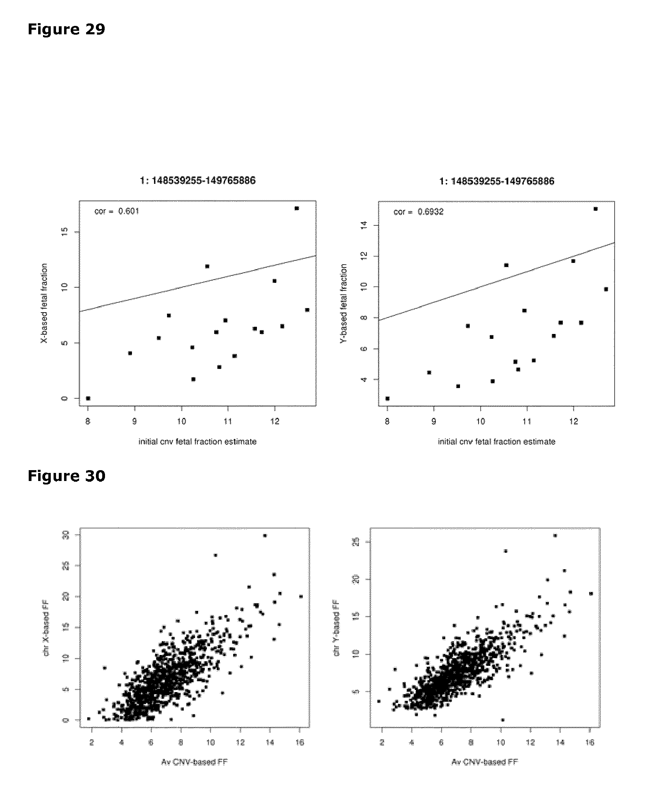

1. CNV 1: 18.39 and 67.96
2. CNV 2: 373.12; 766.69; 1160.26; 1538.69; 1568.97; 1705.20 and 1856.57 (see figure 29).

The normalized counts were extracted for those samples that have normalized counts smaller than the smallest local minimum and larger than 0. It is assumed that the counts for these samples are mainly derived from the fetal DNA and have minimal to low contribution from the maternal DNA.

Based on the normalized counts, the expected count was computed for each CNV. The normalized counts were normalized towards an arbitrary chosen value of 10,000,000 reads.

With this expected count, an estimate of the fetal fractions was computed as:

$$2 \times 100 \times observed\ counts/expected\ counts.$$

This estimate of the fetal fraction is correlated with the actual fetal fraction. However, it was found to be not identical. In fact, the estimated fetal fraction can be seen as:

$$estimated\ fetal\ fraction = factor * actual\ fetal\ fraction.$$

This factor is a constant factor that can be considered as an attribute of each CNV in the dataset, and which is to be empirically determined (see further). Examples of determining fetal fraction:

a) for CNV 1:

Fetal fraction can be estimated as

$$2*100*normalized\ count/expected\ counts = 2*100*normalized\ count\ /88.58$$

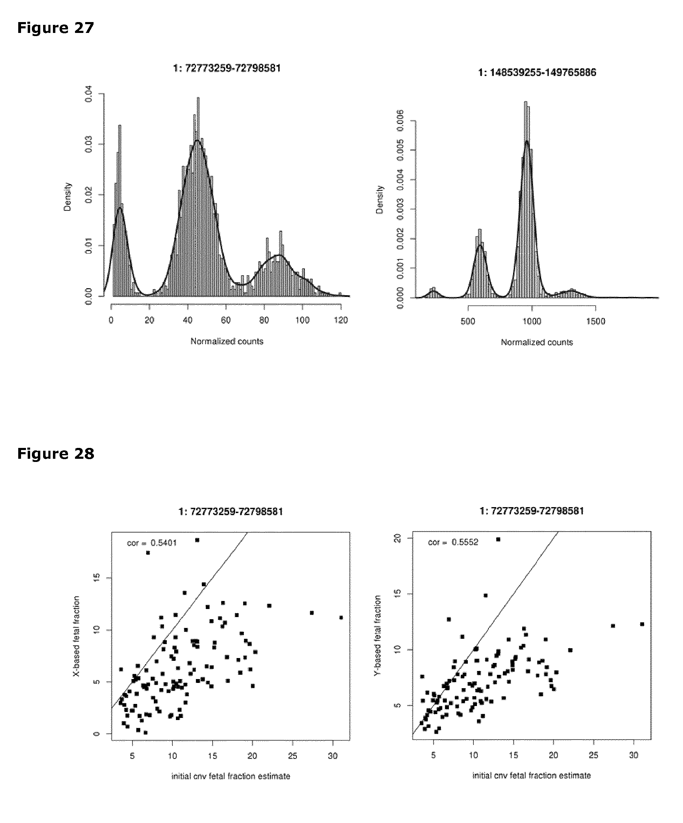

Figure 28 shows the computed fetal fractions for all male pregnancies with less than 18.54 counts for CNV 1 (i.e. 142 samples) versus the in-house fetal fraction computed via chromosome X (at the left) or chromosome Y (at the right). Correlations equal 0.54 and 0.56 with the X and Y-based fetal fractions respectively.

b) for CNV 2

Fetal fraction can be estimated as:

$$2*100*normalized\ count/expected\ counts = 2*100*normalized\ counts/4{,}290$$

Figure 29 shows the computed fetal fractions for all male pregnancies with less than 359.20 counts for CNV 2 (i.e., 17 samples) versus the fetal fraction computed via chromosome X (at the left) or chromosome Y (at the right). Correlations equal 0.601 and 0.963.

For all CNVs the local minimum, the local maximum, number of peaks, number of male pregnancies with a count lower than the smallest local minimum and the correlations with the X/Y-based fetal fractions was computed.

Based on this information, autosomal "pseudo-informative" CNVs were selected

- X or Y-based correlation larger than A (between 0.01 and 1, A was set as 0.5 in this example)
- based on at least B (between 1 and 100 or more, B was set as 8 in this example) male pregnancies
- having more than C peaks (between 0 and 5, C was set as 2) .
- exclude the CNVs on the X and Y chromosome.
- Compare the first local minimum with the third local maximum. The ratio between the third local maximum and the first local minimum should be larger than D (between 0.1 and 100, D is set to 3).

This can strongly reduce the number of CNVs to anywhere between 1 and 100,000 or more "pseudo-informative" CNVs. In the current example about 5,000 "pseudo-informative" CNVs were identified.

Within the list of obtained pseudo-informative CNVs, a lot of overlapping CNVs were identified. The list was therefore cleaned by one or more of the following methodologies:

- Per set of overlapping CNVs, retain only A (A = between 1 and 100 or more, whereby A was set as 1 in this example) of them, i.e. the one with the highest average correlation (i.e., average of the X and Y based correlations.
- Removal of duplicates
- For very similar CNVs, only the longest CNV was retained

Note that a cleanup is optional but can strongly reduce the number of CNVs to anywhere between 1 and 100,000 or more "informative" CNVs. In the current example the number was reduced to about 100 "informative" CNVs.

In a subsequent step and for each of the informative CNVs, the normalized counts were scaled towards the X/Y based fetal fractions. As such, for each CNV it was evaluated how the read counts predict the fetal fraction. The method restricted to CNVs with a correlation above D (whereby D is between 0.01 and 1, and set as 0.5 in the current example). In case the X-based correlation was above D, while the Y-based correlation was below D, then only the X-based fetal fractions were taken into account. Note that the inverse is also possible.

As an example, CNV 1: 1,541,063-1,541,536 was considered. A regression line of the X(Y) based fetal fractions versus the normalized counts was fitted for the small counts seen in male pregnancies of succeeded experiments. This resulted in 2 regression lines, each with an intercept and slope, for all CNVs or all pseudo-informative CNVs, or all informative CNVs.

With the slope and intercept of these regression lines, for each CNV the corresponding, small normalized counts were scaled. Note that this scaling can be done for all samples (i.e., male and female samples). The following plot shows the scaled counts versus the X/Y based fetal fractions for the male pregnancies that had small counts for CNV 1: 1,541,063-1,541,536.

For each CNV and for all samples with small counts for that particular CNV two estimates of the fetal fraction have been obtained, the X and Y based fetal fraction. As third estimate, the average of the X and Y-based scaled counts is taken.

In case one of the 2 correlations, e.g. the X-based correlation, falls below E (whereby E is between 0.01 and 1, and set as 0.5 in the current example E = 0.5 in this example), then the average will be equal to the Y-based scaled counts.

For each sample three estimates for the fetal fraction for all CNVs which show a small number of reads within the sample are obtained:

- X based estimate
- Y based estimate
- averaged-scaled estimate

In a final step, the average of the CNV-based fetal fraction over all CNVs was obtained. In order to check whether this average reflects the chromosome X and Y-based fetal fractions for the male pregnancies, they were plotted versus each other for the succeeded experiments.

| Corr | Av CNV FF | X-scaled CNV FF | Y-scaled CNV FF |
|---|---|---|---|
| Chr-X FF | 0.7774543 | 0.7785994 | 0.7632756 |
| Chr-Y FF | 0.7988806 | 0.7963122 | 0.8066906 |

From figure 31 it is apparent that there is a clear correlation between the CNV based fetal fraction determination and the X and Y based fetal fraction determination. In order to use the CNV based fetal fraction method to estimate the actual fetal fraction, the scaling can be empirically adapted (i.e. the regression line can be adjusted to have a slope of 1).

## Claims

1. A method for determining the presence or absence of a fetal chromosomal aneuploidy in a pregnant female, the method comprising:

   - providing the sequences of at least a portion of the nucleic acid molecules contained in a biological sample obtained from said pregnant female, said biological sample comprises both maternal and fetal cell-free DNA;
   - aligning said obtained sequences to a reference genome;
   - counting the number of reads on a set of chromosomal segments and/or chromosomes thereby obtaining read counts;
   - normalizing said read counts or a derivative thereof into a normalized number of reads;
   - obtaining a first score of said normalized reads and obtaining a collection of scores of said normalized reads, whereby said first score is derived from the normalized reads for a target chromosome or chromosomal segment and whereby said collection of scores is a set of scores derived from the normalized number of reads for a set of chromosomes or chromosome segments that include said target chromosomal segment or chromosome;
   - calculating a parameter p from said first score and said collection of scores, whereby said parameter represents a ratio between
   * said first score, corrected by a summary statistic of said collection of scores, and
   * a summary statistic of said collection of scores; and
   - comparing said parameter p by a cutoff value, whereby said cutoff value is prerequisite for the presence or absence of an aneuploidy of the target chromosome or chromosomal segment.

2. The method according to claim 1, **characterized in that** said number of reads are recalibrated to correct for GC content and/or total number of reads obtained from said sample.

3. The method according to claim 1 or 2, **characterized in that** said normalization occurs via comparison with data obtained from the corresponding chromosomal segments or chromosome from a reference set.

4. The method according to any one of the claims 1 to 3, **characterized in that** said summary statistic is the mean, median, standard deviation, mean absolute deviation or the median absolute deviation.

5. The method according to anyone of the claims 1 to 4, wherein the sequencing is performed randomly on a portion of the nucleic acid molecules contained in the biological sample.

6. The method according to any one of the previous claims, wherein the biological sample is maternal blood, plasma, serum, urine, blastocoel fluid, transcervical fluid or saliva.

7. The method according to anyone of the previous claims, wherein the target chromosomal segment is selected from Table 1, and/or from a bin or a window derived from chromosome X, Y, 6, 7, 8, 13, 14, 15, 16, 18, 21 and/or 22.

8. The method according to anyone of the previous claims 1 to 6, **characterized in that** said target chromosome is selected from chromosome X, Y, 6, 7, 8, 13, 14, 15, 16, 18, 21 and/or 22.

9. The method according to any one of the previous claims, wherein said cutoff value is established using standard statistical considerations, or empirically established by using biological samples.

10. The method according to any one of the previous claims whereby said score is calculated as:

$$Zi = \frac{GRi - \mu\,ref,i}{\sigma\,ref,i}\text{ ,}$$ whereby i is a chromosome or chromosomal segment or the target chromosome or target chromosomal segment.

11. The method according to claim 10, **characterized in that** said parameter p is calculated as:

$$Z \text{of } Z_i = \frac{Z_i - \underset{j=i,a,b,\ldots}{\text{median}}(Z_j)}{\underset{j=i,a,b,\ldots}{\text{mad}}(Z_j)}$$

whereby (Zj) represents a collection of scores that are derived from chromosomes or chromosomal segments i, a, b, .. whereby i corresponds to the target chromosomal segment or chromosome;

12. The method according to any of the previous claims, comprising the calculation of secondary parameters, whereby said secondary parameters are a prerequisite of the presence of said aneuploidy and/or a measure of the quality of the sample.

13. The method according to claim 10, whereby said secondary parameters are compared to a cutoff value.

14. The method according to any one of the claims 12 or 13, whereby said presence or absence of an aneuploidy is determined by the comparison of said parameter to a cutoff value and the comparison of one or more secondary parameters to corresponding cutoff values.

15. The method according to any one of the previous claims, wherein the fetal fraction of the sample is determined.

16. The method according to claim 15, whereby said determination of fetal fraction comprises the steps of:

- counting the number of sequences that align to a predefined set of polymorphisms;
- comparing the obtained number of sequences with the expected number of sequences to identify the informative polymorphic site(s) for the sample;
- calculating from the obtained number of sequences for said informative polymorphic site(s) an amount, whereby said amount is an indication for the fetal fraction.

17. The method according to claim 16, whereby said amount is calculated using linear scaling based on informative polymorphism-specific attributes.

18. The method according to anyone of the previous claims 15 to 17, whereby said amount indicative for the fetal fraction serves as a quality control of said sample.

19. A computer program product comprising a computer readable medium encoded with a plurality of instructions for controlling a computing system to perform an operation for performing prenatal diagnosis of a fetal chromosomal aneuploidy in a biological sample obtained from a pregnant female subject, wherein the biological sample includes nucleic acid molecules, the operation comprising the steps of

- receiving the sequences of at least a portion of the nucleic acid molecules contained in a biological sample obtained from said pregnant female, said biological sample comprises both maternal and fetal cell-free DNA;
- aligning said obtained sequences to a reference genome;
- counting the number of reads on a set of chromosomal segments and/or chromosomes thereby obtaining read counts;

- normalizing said number of reads or a derivative thereof into a normalized number of reads;
- obtaining a first score of said normalized reads and a collection of scores of said normalized reads, whereby said first score is derived from the normalized reads for a target chromosome or chromosomal segment and whereby said collection of scores is a set of scores derived from the normalized reads for a set of chromosomes or chromosomal segments that include said target chromosomal segment or chromosome;
- calculating a parameter p from said first score and said collection of scores, whereby said parameter represents a ratio between
* said first score, corrected by a summary statistic of said collection of scores, and
* a summary statistic of said collection of scores; and
- comparing said parameter p by a cutoff value, whereby said cutoff value is prerequisite for the presence or absence of an aneuploidy of the target chromosome or chromosomal segment.

20. Computer program product according to claim 19, further comprising operations for calculating one or more secondary parameters, whereby said secondary parameters are a prerequisite of the presence of said aneuploidy and/or a measure of quality of the sample.

21. Computer program product according to any one of the previous claims, comprising operations for determining the fetal fraction.

22. Computer program product according to any one of the previous claims, further comprising operations for performing CNV calling, CNV quantification and/or CNV signature recognition.

23. A kit comprising a computer program product according to any one of the claims 19 to 22 and a protocol for obtaining the sequences of at least a portion the nucleic acid molecules contained in a biological sample obtained from a pregnant female, said biological sample comprises both maternal and fetal cell-free DNA.

24. Kit according to claim 23, further comprising reagents and means for obtaining said sequences.

25. A report, comprising an estimation of the presence or absence of a fetal chromosomal aneuploidy in a pregnant female, said report comprises the parameter, one or more secondary parameters and comparison to a cutoff value as defined in any one of the claims 1 to 18 and a visualization of said reads per chromosome.

26. Report according to claim 25, **characterized in that** said visualization depicts said first score per window of a target chromosome and/or parameter p.

27. A method for determining a fetal fraction in a biological sample obtained from a pregnant female, said method comprises:

- receiving the sequences of at least a portion of the nucleic acid molecules contained in a biological sample obtained from said pregnant female;
- counting the number of sequences that align to a predefined set of polymorphisms;
- comparing the obtained number of sequences with the expected number of sequences to identify the informative polymorphic site(s) for the sample;
- calculating from the obtained number of sequences for said informative polymorphic site(s) an amount, whereby said amount is an indication for the fetal fraction.

28. The method according to claim 27, whereby said amount is calculated using linear scaling based on informative polymorphism-specific attributes.

29. Method according to claim 27 or 28, **characterized in that** said polymorphisms are copy number variations with a size between 100 bp and 1 Mb, or between 1 kb and 1 Mb, or between 2 bp and 250 Mb.

30. Computer program product for comprising a computer readable medium encoded with a plurality of instructions for controlling a computing system to perform an operation of determining or estimating the fetal fraction in a biological sample obtained from a pregnant female subject, wherein the biological sample includes nucleic acid molecules, the operation comprising the steps of:

- receiving the sequences of at least a portion of the nucleic acid molecules contained in a biological sample obtained from said pregnant female;
- counting the number of sequences that align to a predefined set of polymorphisms
- comparing the obtained number of sequences with the expected number of sequences to identify the informative polymorphic site(s) for the sample; and
- calculating from the obtained number of sequences for said informative polymorphic site(s) an amount, whereby said amount is an indication for the fetal fraction.

**Figure 1**

**Figure 2**

**Figure 3**

**Figure 4**

**Sample A: BM scores**

**Figure 5**

**Figure 6**

Sample B: ZZ2 scores

**Figure 7**

**Figure 8**

Sample B: BM scores

**Figure 9**

**Figure 10**

Sample A: ZofZ scores

**Figure 11**

**Figure 12**

**Figure 13**

Sample B: ZofZ scores

**Figure 14**

**Figure 15**

Sample A: OM scores

**Figure 16**

Sample B: OM scores

**Figure 17**

**Figure 18**

**Figure 19**

**Figure 20**

**Figure 21**

**Figure 22**

**Figure 23**

**Figure 24**

**Figure 25**

**Figure 26**

**Figure 27**

**Figure 28**

**Figure 29**

**Figure 30**

Europäisches
Patentamt

European
Patent Office

Office européen
des brevets

# EUROPEAN SEARCH REPORT

**Application Number**

EP 15 17 6401

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/209597 A2 (ARIOSA DIAGNOSTICS INC [US]) 31 December 2014 (2014-12-31)<br>* abstract *<br>* page 6, paragraph 18 *<br>* page 2, paragraph 5 - paragraph 6 *<br>* page 3, paragraph 8 - paragraph 9 *<br>* page 4, paragraph 12 *<br>* page 6, paragraph 19 - page 7 *<br>* page 7, paragraph 20 - page 8, paragraphs 21,22 *<br>* page 36, paragraph 104 - page 37, paragraph 105 *<br>* page 43, paragraph 122 *<br>----- | 1-30 | INV.<br>C12Q1/68 |
| X | WO 2011/102998 A2 (HELICOS BIOSCIENCES CORP [US]; LAPIDUS STANLEY N [US]; THOMPSON JOHN F)<br>25 August 2011 (2011-08-25)<br>* abstract *<br>* page 2 *<br>* page 20 *<br>* page 22, last paragraph *<br>* page 27 *<br>* page 29 *<br>* page 31, paragraph 5 - paragraph 6 *<br>* page 32 - page 33 *<br>* page 33, line 15 - line 19 *<br>----- | 1-30 | **TECHNICAL FIELDS SEARCHED (IPC)**<br><br>C12Q |

The present search report has been drawn up for all claims

| Place of search | Date of completion of the search | Examiner |
|---|---|---|
| Munich | 15 January 2016 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 15 17 6401

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2016

| Patent document cited in search report | | Publication date | Patent family member(s) | | Publication date |
|---|---|---|---|---|---|
| WO 2014209597 | A2 | 31-12-2014 | US | 2015004601 A1 | 01-01-2015 |
| | | | WO | 2014209597 A2 | 31-12-2014 |
| WO 2011102998 | A2 | 25-08-2011 | AU | 2011218382 A1 | 11-10-2012 |
| | | | AU | 2015246128 A1 | 12-11-2015 |
| | | | CN | 103108960 A | 15-05-2013 |
| | | | EP | 2536852 A2 | 26-12-2012 |
| | | | US | 2010216153 A1 | 26-08-2010 |
| | | | US | 2013022977 A1 | 24-01-2013 |
| | | | US | 2014322709 A1 | 30-10-2014 |
| | | | WO | 2011102998 A2 | 25-08-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• EP 2183693 A

• US 8195415 B

**Non-patent literature cited in the description**

• **BENJAMINI et al.** *Nucleic Acid Research,* 2012